# FASCICULE DE BREVET EUROPEEN

(11) **EP 1 809 754 B1**
(45) Date de publication et mention de la délivrance du brevet: **11.02.2015**
(21) Numéro de dépôt: 05809308.9
(22) Date de dépôt: 11.10.2005
(51) Int. Cl.: C12N 15/53, C12P 3/00, C12N 9/02, C12N 1/13

(54) **PRODUCTION D'HYDROGENE PAR EXPRESSION HETEROLOGUE D'UNE NAD (P) H DESHYDROGENASE DE TYPE II CHEZ CHLAMYDOMONAS.**
HERSTELLUNG VON WASSERSTOFF DURCH HETEROLOGE EXPRESSION EINER TYP-II-NAD(P)H-DEHYDROGENASE IN CHLAMYDOMONAS
PRODUCING HYDROGEN BY HETEROLOGOUS EXPRESSION OF A TYPE II NAD (P) H DEHYDROGENASE IN CHLAMYDOMONAS

(30) Priorité: 11.10.2004 FR 0410715
(43) Date de publication de la demande: 25.07.2007
(73) Titulaire: Commissariat à l'Énergie Atomique et aux Énergies Alternatives, 75015 Paris (FR)
(72) Inventeur: BERNARD, Laetitia, 83700 SAINT-RAPHAËL (FR); MUS, Florence, 84170 MONTEUX (FR); SIMON-DESPLATS, Carine, F-83500 LA SEYNE-SUR-MER (FR); CUINE, Stéphan, F-04100 MANOSQUE (FR); COURNAC, Laurent, F-84240 LA TOUR D'AIGUES (FR); PELTIER, Gilles, F-04860 PIERREVERT (FR)
(74) Mandataire: Rançon, Xavier Lucien Abel
(86) Numéro de dépôt international: PCT/FR2005/002511
(87) Numéro de publication internationale: WO 2006/040471

(56) Documents cités:
- WO-A-03/067213
- US-A- 4 532 210
- US-A1- 2001 053 543
- L. COURMAC ET AL: "Limiting steps of hydrogen production in Chlamydomonas reinhardtii and Synechocystis PCC 6803 as analysed by light-induced gas exchange transients" INTERNATIONAL JOURNAL OF HYDROGEN ENERGY, vol. 27, 2002, pages 1229-1237, XP002329973 cité dans la demande
- COURNAC L ET AL: "Interactions between chlororespiration, photosynthesis, and hydrogen production in Chlamydomonas reinhardtii" PHOTOSYNTHESIS RESEARCH, vol. 69, no. 1-3, 2001, page 257, XP002329974 & 12TH INTERNATIONAL CONGRESS ON PHOTOSYNTHESIS; BRISBANE, AUSTRALIA; AUGUST 18-23, 2001 ISSN: 0166-8595
- PELTIER GILLES ET AL: "Chlororespiration." ANNUAL REVIEW OF PLANT BIOLOGY. VOLUME 53 ANNUAL REVIEWS, 4139 EL CAMINO WAY, P. O. BOX 10139, PALO ALTO, CA, 94303-0139, USA SERIES : ANNUAL REVIEW OF PLANT BIOLOGY, 2002, pages 523-550, XP002329975 ISSN: 0-8243-0653-8 cité dans la demande
- DATABASE EMBL 5 juillet 2004 (2004-07-05), B. GOODNER ET AL: "AGR_C_3667p" XP002329976 extrait de EBI, HINXTON, UK Database accession no. Q7CY19 & B. GOODNER ET AL: "genome sequence of the plant pathogen and biotechnology agent Agrobacterium tumefaciens C58." SCIENCE, vol. 294, 2001, pages 2323-2328,
- MUS F ET AL: "Inhibitor studies on non-photochemical plastoquinone reduction and H2 photoproduction in Chlamydomonas reinhardtii" BIOCHIMICA ET BIOPHYSICA ACTA. BIOENERGETICS, AMSTERDAM, NL, vol. 1708, no. 3, 26 mai 2005 (2005-05-26), pages 322-332, XP004971733 ISSN: 0005-2728

## Description

La présente invention est relative à l'amélioration de la production d'hydrogène par *Chlamydomonas reinhardtii.*

L'hydrogène est une matière première essentielle de l'industrie chimique, et constitue également un combustible appelé à jouer un rôle majeur dans les décennies à venir. Les piles à combustibles alimentées par de l'hydrogène permettent, par réaction de l'hydrogène avec l'oxygène de l'air, de produire de l'électricité de manière non-polluante, en ne rejetant que de la vapeur d'eau. Les avancées technologiques dans le domaine des piles à combustibles rendent leur utilisation à grande échelle de plus en plus envisageable.

Toutefois, la majeure partie de l'hydrogène actuellement utilisé est produit à partir de sources d'énergie fossiles, telles que le pétrole ou le charbon, par des techniques elles-mêmes génératrices de pollution, telles que la conversion catalytique des hydrocarbures du gaz naturel ou le craquage du pétrole ou du charbon.

Il apparaît donc souhaitable de disposer de procédés rentables permettant la production d'hydrogène à partir d'une source d'énergie primaire renouvelable et propre (non libératrice de gaz à effet de serre).

Certaines algues vertes unicellulaires appartenant aux genres *Scenedesmus*, *Chlorococcum, Chlorelle* (ordre des Chlorococcales), *Lobochlamys* et *Chlamydomonas* (ordre des Volvocales), telles que l'espèce *Chlamydomonas reinhardtii,* sont capables de produire de l'hydrogène à partir d'énergie solaire, en utilisant l'eau comme donneur d'électrons et de protons.

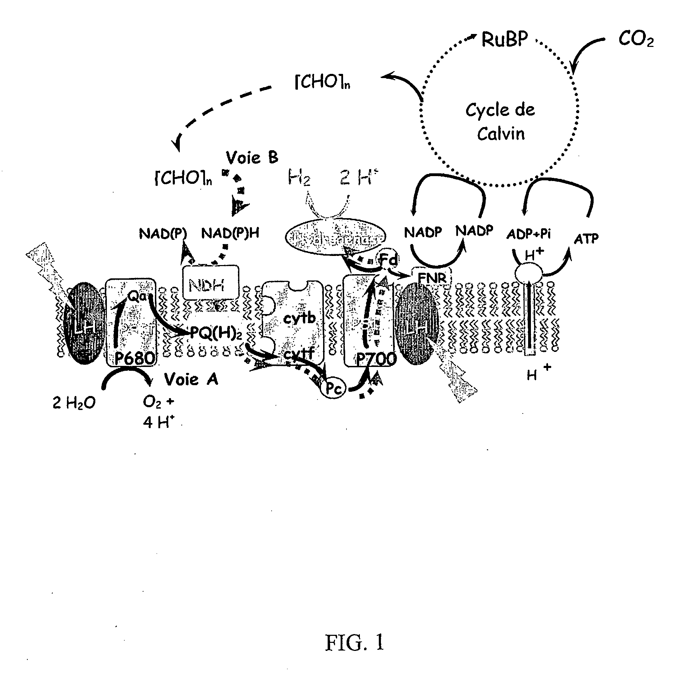

Chez ces algues, l'hydrogène est produit par une hydrogénase à fer, ayant une forte activité spécifique. Cette enzyme est connectée à la chaîne photosynthétique de transfert d'électrons PSI *via* un transporteur d'électrons commun, la ferrédoxine. Les électrons nécessaires à la production d'hydrogène peuvent être fournis au PSI soit par l'activité du PSII (voie A), soit par l'utilisation des réserves carbonées *via* la réduction non-photochimique des plastoquinones (voie B). Ces deux voies sont schématisées sur la Figure 1.

Légende de la Figure 1 : En traits pleins, la voie A, PSII-dépendante ; en pointillés la voie B, reposant sur une réduction des plastoquinones, indépendante du PSII.

PSI : Photosystème I ; PSII : Photosystème II ; RuBP : ribulose 1,5 bisphosphate ; LHC : light harvesting complex ; FNR : ferrédoxine NADP réductase ; Fd : ferrédoxine ; Pc :plastocyanine ; cytb6 : cytochrome b6 ; cytf : cytochrome f ; NDH : NADH-deshydrogénase ; PQ(H)₂ : plastoquinol ; Qa : quinone a ; P680 et P700 : centres réactionnels des PSI et PSII, respectivement.

En conditions naturelles, la production d'H₂ n'est qu'un phénomène transitoire. En effet, l'hydrogénase est très sensible à l'O₂. Or, la photolyse de l'eau intervenant au niveau du photosystème II, qui fournit des électrons pour la production d'H₂ par la voie A, produit également de l'O₂ qui induit une inhibition rapide de l'hydrogénase.

Diverses solutions ont été proposées pour remédier à ce problème. La première solution repose sur une production à l'obscurité, les autres sur une production à la lumière utilisant en partie la voie B, qui, contrairement à la voie A, ne conduit pas à une production d'oxygène.

Par exemple, le Brevet U.S. 4,532,210 décrit un procédé alternant des phases de lumière et d'obscurité. Au cours des périodes lumineuses, les algues produisent de l'O₂ et accumulent des réserves hydrocarbonées produites par photosynthèse. Ces réserves sont ensuite utilisées en anaérobie pendant les phases obscures pour produire de l'hydrogène. Cette méthode nécessite une purge à l'azote pour atteindre l'anaérobie. Elle est en outre limitée par l'efficacité de la production d'H₂ à l'obscurité, qui est inférieure d'un ordre de grandeur à la production à la lumière.

La Demande U.S. 2001/0053543 décrit un procédé basé sur l'inhibition réversible du photosystème II par une carence en soufre. Ce procédé comprend une étape de culture des algues vertes, à la lumière, et dans un milieu à teneur normale en soufre, pour permettre l'accumulation de réserves hydrocarbonées, et une étape de culture en récipients scellés et à la lumière, dans un milieu dépourvu de soufre. L'inhibition du photosystème II entraîne la cessation de la production d'oxygène par la photosynthèse. Lorsque les algues (dont la respiration n'est pas inhibée par la carence en soufre) ont utilisé la totalité de l'oxygène présent dans le milieu, elles deviennent anaérobies et utilisent les réserves hydrocarbonées constituées grâce à la photosynthèse pour produire de l'H₂. L'alternance de phases de culture en présence et en absence de soufre permet une séparation temporelle des phases lumineuses de production d'O₂ et d'H₂.

La Demande U.S. 2003/0162273 propose une méthode alternative pour induire une carence en soufre inhibant le photosystème II ; il s'agit de l'utilisation d'une algue génétiquement modifiée sous-exprimant une sulfate perméase chloroplastique.

Les méthodes décrites ci-dessus permettent d'éviter l'inhibition de l'hydrogénase à fer, en séparant la production d'O₂ et celle d'H₂. Cependant, la production d'hydrogène par les trois méthodes décrites ci-dessus présente des limitations. La première méthode repose sur l'activité fermentaire de l'algue à l'obscurité, phénomène peu efficace ne conduisant qu'à une production d'hydrogène marginale. Les seconde et troisième méthodes reposent sur le fonctionnement parallèle des voies A et B. La voie A s'accompagnant d'un dégagement d'oxygène, doit être maintenue à un niveau inférieur à la consommation d'O₂ respiratoire afin de maintenir l'anoxie. La voie A est donc limitée par la capacité respiratoire des algues. La contribution de la voie B est significative mais limitée.

La présente invention a pour but d'améliorer le rendement de cette deuxième voie (B). Dans ce but, les Inventeurs ont eu l'idée d'utiliser dans le chloroplaste une NADH-déshydrogénase de type II pour stimuler la réaction de réduction des plastoquinones.

Les NADH-déshydrogénases de type I et II sont des enzymes capables de réduire les quinones des chaînes de transfert d'électrons. Elles sont associées aux chaînes respiratoires mitochondriales et bactériennes (KERSCHER, Biochim. Biophys. Acta 1459: 274-283, 2000).

Les NADH déshydrogénases de type I (NDH-I) sont des complexes multimériques transmembranaires comprenant de 14 à environ 50 sous-unités. Ce type de complexe n'oxyde que le NADH et présente une activité associée de pompage de protons.

Les NAD(P)H déshydrogénases de type II (NDH-II) sont des enzymes monomériques de type oxydoréductase, de poids moléculaire compris entre 30 et 60 kDa capables de réduire les quinones des chaînes respiratoires bactériennes ou des chaînes mitochondriales des plantes et des levures, en oxydant le NADH ou le NADPH. Leur association avec les chaînes photosynthétiques des plantes et des algues a également été proposée mais n'a pas été démontrée à ce jour. Ce type d'enzyme n'a pas été mis en évidence dans le règne animal.

Dans les chloroplastes des plantes supérieures, l'existence d'un complexe NDH-I fonctionnel a été démontrée (BURROWS et al., EMBO J. 17: 868-876, 1998; SAZANOV et al., Proc. Natl. Acad. Sci. USA 95: 1319-1324, 1998; HORVATH et al., Plant Physiol. 123: 1337-1349, 2000) et l'existence d'une activité de type NDH-II a également été proposée (CORNEILLE et al., Biochim. Biophys. Acta 1363: 59-69, 1998). Chez *Chlamydomonas reinhardtii,* les gènes codant pour le complexe NDH-I chloroplastique sont absents. Toutefois, l'existence d'une activité de type NDH-II a été suggérée (COURNAC et al., Int. J. Hydrog. Energy 27: 1229-1237, 2002; PELTIER et COURNAC, Annu. Rev. Plant Biol. 53: 523-550,2002).

En particulier, COURNAC et al. (Int. J. Hydrog. Energy 27: 1229-1237, 2002) s'intéresse à la production d'hydrogène chez *Chlamydomonas reinhardtii* et chez la cyanobactérie *Synechocystis.* Les auteurs ont étudié le système de transfert d'électrons et ont mis en évidence chez *Chlamydomonas* que la réduction non-photochimique des plastoquinones nécessite une NAD(P)H-PQ oxydoréductase qui doit être une NADH de type-II.

La présente invention est définie aux revendications 1 à 5 ci-après.

Les inventeurs décrivent l'utilisation d'une NAD(P)H déshydrogénase de type II (NDH-II), ou d'un polynucléotide codant pour ladite protéine, pour augmenter la capacité d'une algue verte à produire de l'hydrogène.

Selon un mode de réalisation préféré ladite algue verte est une algue verte unicellulaire, choisie de préférence parmi les Chlorococcales, notamment les genres *Scenedesmus, Chlorococcum, Chlorella,* et les Volvocales, notamment les genres *Lobochlamys* et *Chlamydomonas.*

Selon une disposition préférée de ce mode de réalisation, ladite algue appartient au genre *Chlamydomonas.* Avantageusement, ladite algue appartient à l'espèce *Chlamydomonas reinhardtii.*

On définit comme « NDH-II » toute flavoenzyme possédant :
a) des caractéristiques communes à l'ensemble des NAD(P)H déshydrogénases, à savoir i) la capacité à catalyser la réduction des quinones des chaînes de transfert d'électrons par l'oxydation du NADH ou du NAD(P)H, en utilisant le FAD ou le FMN comme cofacteur flavinique, et ii) la présence dans sa séquence, d'au moins un exemplaire du motif consensus GxGxxG où « G » représente une glycine et « x » représente un acide aminé quelconque, qui correspond au site de fixation du cofacteur flavinique et du NAD(P)H ;
b) des caractéristiques spécifiques aux NAD(P)H déshydrogénases de type II, à savoir l'activité sous la forme d'un monomère de 30 à 60 kDa ou d'un homodimère, le fait de ne pas réaliser de transfert transmembranaire de protons, et de posséder une activité insensible à la roténone.

Pour une revue détaillée sur les NDH-II, on peut notamment se reporter aux revues de YAGI (J. Bioenergetics Biomembranes 23 : 211-224, 1991), de KERSCHER (Biochim. Biophys. Acta 1459 : 274-283, 2000), et de MELO et al. (Microbiol Mol Biol Rev. 68 : 603-616, 2004).

Les Inventeurs ont ainsi utilisé la NDH-I I *d'Agrobacterium tumefaciens* (N° d'accès NCBI: AI2824 ; N° d'accès SWISSPROT: Q8UDU6), également dénommée ci-après Agtundh2. La séquence nucléotidique codant pour cette enzyme est représentée dans la liste de séquences en annexe sous le numéro SEQ ID NO: 1, et la séquence polypeptidique déduite sous le numéro SEQ ID NO: 2.

D'autres NDH-II pouvant être utilisées pour la mise en oeuvre de la présente invention sont à titre d'exemples non-limitatifs, les NDH-II d'*Acidianus ambivalens* (AJ489504), de *Corynebacterium glutamicum* (CAB41413.1), d'*Escherichia coli* (NP_415627), de *Synechocystis sp.* (HOWITT et al., J. Bacteriol. 181(13): 3994-4003, 1999; ORF slr1743 : BAA17783), de *Zymomonas mobilis* (AAD56918), de *Bacillus subtilis* (NP_389111), d'*Azotobacter vinelandi* (AAK19737), de *Trypanosoma brucei* (AAM95239.1), de *Solanum tuberosum* (CAB52796.1, CAB52797.1), de *Saccharomyces cerevisiae* (YML120C (NP_013586), YMR145c (NP_013865.1), YDL085w (NP_010198.1)), de *Neurospora crassa* (CAB41986, EAA27430), de *Yarrowia lipolytica* (XP_505856).

Avantageusement on peut également utiliser une NDH-II endogène de *Chlamydomonas reinhardtii.* Des séquences codant des NDH-II putatives ont été identifiées dans la séquence complète du génome de *Chlamydomonas reinhardtii* (version 2) sur le site « http://genome jgi-psf.org/chlre2/chlre2.home.html », sous les identifications C_310108, C_1170009, C_5950001, C_1890016, C_1450028, C_1450029, C_270109. A partir de ces séquences, les Inventeurs ont identifié une séquence codant effectivement pour une NDH-II. Cette séquence, dénommée ci-après *N2Cr2*, est représentée dans la liste de séquences en annexe sous le numéro SEQ ID NO: 3, et la séquence polypeptidique déduite, dénommée N2Cr2, est représentée sous le numéro SEQ ID NO: 4.

Les Inventeurs ont cloné et exprimé un fragment de *N2Cr2* correspondant aux nucléotides 199-1857 de la séquence SEQ ID NO: 3 (et codant donc pour un polypeptide correspondant aux acides aminés 67-619 de la séquence SEQ ID NO: 4) et ont montré que le polypeptide codé par ce fragment correspondait à une NDH-II à localisation chloroplastique.

La NDH-II N2Cr2, ainsi que tout fragment de cette protéine présentant une activité NAD(P)H déshydrogénase, et les polynucléotides codant pour ladite NDH-II ou ledit fragment font également partie de l'objet de la présente invention.

Un certain nombre des NDH-II mentionnées ci-dessus, présentent une affinité préférentielle pour le NADH. Or, bien que le NADH et le NADPH soient tous deux présents au sein du chloroplaste, c'est le NADPH qui représente la forme majoritaire. Dans le but d'augmenter l'efficacité, dans un contexte chloroplastique, des NDH-II utilisant préférentiellement le NADH, les Inventeurs ont eu l'idée de modifier ces enzymes, pour augmenter leur efficacité pour le NADPH.

Ils ont effectué une mutagenèse dirigée de l'Agtundh2, et ont montré que la substitution du résidu acide (glutamate dans le cas d'Agtundh2) situé, chez les enzymes utilisant préférentiellement le NADPH, à la fin du second feuillet beta du motif beta-alpha-beta de liaison des pyridine nucléotides (également dénommé « motif de Rossman »), par un résidu polaire neutre tel que ceux rencontrés à la même position chez les enzymes utilisant préférentiellement le NADH, permettait d'augmenter son affinité pour le NADPH.

Les inventeurs décrivent une NDH-II mutante, obtenue à partir d'une NDH-II utilisant préférentiellement le NADH, par substitution du résidu glutamate ou aspartate situé à la fin du second feuillet beta du motif beta-alpha-beta de liaison des pyridine nucléotides, par un résidu polaire neutre, préférentiellement un résidu glutamine ou asparagine.

Ce résidu correspond par exemple à la position 201 de la séquence d'Agtundh2 (SEQ ID NO: 2), et à la position 285 de la séquence de N2Cr2 (SEQ ID NO: 4).

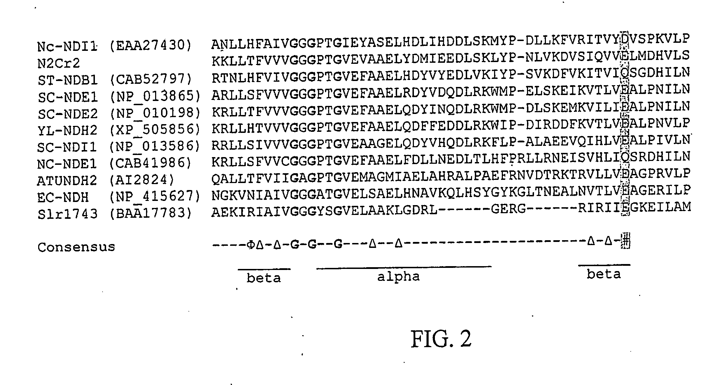

La Figure 2 représente un alignement des séquences de différentes NDH-II, au niveau du site de liaison des pyridine nucléotides. La séquence consensus du motif beta-alpha-beta est représentée au dessous de l'alignement de séquences ; Φ représente un résidu hydrophile ; Δ représente un résidu hydrophobe, et # représente le résidu situé à la fin du second feuillet beta ; il s'agit d'un résidu acide chez les NDH-II utilisant préférentiellement le NADH, et d'un résidu polaire neutre chez les NDH-II qui utilisent préférentiellement le NADPH (par exemple, comme le montre l'alignement de la Figure 2, les NDH-II ST-NDB1 et NC-NDE1 ont un résidu glutamine à cette position).

La présente invention a pour objet un procédé pour accroître la capacité d'une algue verte à produire de l'hydrogène, caractérisé en ce qu'il comprend la transformation génétique de ladite algue par un polynucléotide codant pour une NAD(P)H déshydrogénase de type II (NDH-II) telle que définie ci-dessus, et l'expression de ladite NDH-II dans ladite algue, ladite NDH-II :
- présentant au moins un exemplaire du motif consensus GxGxxG où « G » représente une glycine et « x » représente un acide aminé quelconque,
- ayant la capacité à catalyser la réduction des quinones des chaînes de transfert d'électrons par l'oxydation du NADH ou du NADPH, en utilisant un cofacteur flavinique, et
- étant, dans sa forme active, un monomère de 30 à 60 kDa ou un homodimère.

Pour la mise en oeuvre de la présente invention, on utilisera les techniques usuelles du génie génétique. Classiquement, on construit une cassette d'expression en plaçant un polynucléotide codant pour la NDH-II que l'on souhaite exprimer, sous contrôle de séquences de régulation de l'expression (notamment promoteur et terminateur de transcription) appropriées. Avantageusement, ledit polynucléotide codant pour la NDH-II est fusionné à une séquence d'adressage chloroplastique.

La cassette d'expression peut également comprendre, en outre, des éléments de régulation de la transcription et/ou de la traduction, parmi lesquels on citera notamment les activateurs (enhancers) ou les atténuateurs (silencers) de transcription, les séquences de tête, les séquences de polyadénylation, etc.

La cassette d'expression ainsi obtenue est ensuite insérée dans un vecteur approprié, qui est utilisé pour transformer la cellule ou l'organisme-hôte choisi. Différents outils et méthodes utilisables pour la transformation des algues vertes sont connus en eux-mêmes, et peuvent être utilisés pour la mise en oeuvre de la présente invention (pour revue, ROCHAIX et al., The Molecular Biology of Chloroplasts and Mitochondria in Chlamydomonas, Kluwer Academic Publishers, Netherlands, 1998).

A titre d'exemples non-limitatifs de séquences de régulation de l'expression (promoteurs, terminateurs, etc.), et de séquences d'adressage chloroplastique utilisables dans le cadre de la présente invention, on citera :
- pour une expression nucléaire, les promoteurs, terminateurs et peptides d'adressage des gènes *RbcS2* codant pour la petite sous-unité de la RUBISCO (ribulose biphosphate carboxylase/oxygénase) (GOLDSCHMIDT-CLERMONT et RAHIRE, J. Mol. Biol. 191 : 421-432, 1986), *AtpC* codant pour la sous-unité gamma de l'ATP synthétase chloroplastique (QUINN et MERCHANT, Plant Cell 7: 623-638, 1995; KINDLE et LAWRENCE. Plant Physiol. 116 : 1779-1791, 1998), et *PetE* codant pour la plastocyanine (QUINN et MERCHANT, 1995, précité ; KINDLE, Plant Mol. Biol. 38 : 365-377, 1998), qui peuvent être combinés de façon différente ; et
- pour une expression chloroplastique, les promoteurs, terminateurs et peptides d'adressage des gènes *RbcL* codant pour la grande sous unité de la RUBISCO, *AtpB* codant pour la sous-unité bêta de l'ATP synthétase, et *PsbA* codant pour la sous-unité D1 du photosystème II (BATEMAN et PURTON, Mol. Gen. Genet. 263 : 404-410, 2000).

A titre d'exemples non limitatifs de marqueurs de sélection utilisables dans le cadre de la présente invention, on citera :
- pour une expression nucléaire, les gènes *Arg7* codant pour l'arginosuccinate lyase de *Chlamydomonas* qui complémente un mutant déficient et donc auxotrophe pour l'arginine (DEBUCHY et al., EMBO J. 8 : 2803-2809, 1989), *Nit 1* codant pour la nitrate réductase de *Chlamydomonas* qui complémente un mutant déficient et donc incapable de croître avec des nitrates comme seule source d'azote (KINDLE et al., J. Cell Biol. 109 : 2589-2601, 1989), *Nic7* codant pour une enzyme intervenant dans la biosynthèse du nicotinamide de *Chlamydomonas* qui complémente un mutant déficient et donc auxotrophe pour le nicotinamide (FERRIS, Genetics 141 : 543-549, 1995), *Oeel* codant pour une sous-unité du photosystème II de *Chlamydomonas* qui complémente un mutant strictement hétérotrophe (MAYFIELD et KINDLE, Proc. Natl. Acad. Sci. USA. 87 : 2087-2091, 1990), *aadA* codant pour l'aminoglycoside adénine transférase d'*E*. *coli* qui confère la résistance à la spectinomycine et la streptomycine à n'importe quelle souche de *Chlamydomonas* (CERUTTI et al., Genetics. 145 : 97-110, 1997), *ble* codant pour une protéine de fixation à la bléomycine de *Streptoalloteichus hindustanus* qui confère la résistance à la phléomycine à n'importe quelle souche de *Chlamydomonas* (STEVENS et al., Mol. Gen. Genet. 251 : 23-30, 1996) ; les gènes codant pour des résistances aux antibiotiques doivent être exprimés sous le contrôle de séquences régulatrices de *Chlamydomonas* comme celles du *RbcS2 ;* et
- pour une expression chloroplastique, la complémentation d'un mutant obtenu par délétion ou insertion dans un gène codant pour une protéine de la chaîne photosynthétique par le gène natif porté par le fragment d'ADN inséré, par exemple le gène *psbH* codant pour une sous-unité du PSII (BATEMAN et PURTON,. Mol. Gen. Genet. 263 : 404-410, 2000), et les gènes *aadA* codant pour l'aminoglycoside adénine transférase d'E. *coli* qui confère la résistance à la streptomycine et la spectinomycine, et *aphA-6* codant pour l'aminoglycoside phosphotransférase *d'Acinetobacter baumanii* qui confère la résistance à la kanamycine et l'amikacine.

La transformation des algues vertes peut être réalisée par diverses méthodes telles que, par exemple l'insertion d'un ADN dans le génome nucléaire par des transformations par billes de verre (KINDLE, Proc. Natl. Acad. Sci. USA. 87 : 1228-1232, 1990), par biolistique (DEBUCHY et al., EMBO J. 8 : 2803-2809, 1989), et par électroporation (SHIMOGAWARA et al., Genetics. 148 : 1821-1828, 1998).

Pour la transformation de l'ADN chloroplastique, et l'expression dans les chloroplastes, on utilisera avantageusement l'insertion par recombinaison homologue d'un ADN encadré par des séquences homologues à celle du génome chloroplastique et correspondant à des zones neutres du génome (non codantes et non régulatrices), ainsi que les techniques de transformation précédemment citées pour l'insertion dans le génome nucléaire, en particulier la biolistique qui donne les meilleurs rendements.

L'invention concerne également les algues vertes transformées par un polynucléotide codant pour une NDH-II telle que définie ci-dessus.

Les algues vertes conformes à l'invention peuvent être utilisées pour la production d'hydrogène, dans les mêmes conditions que les algues vertes non-transformées. Elles peuvent par exemple être utilisées dans le cadre de procédés tels que ceux décrits dans le Brevet U.S. 4,532,210 ou la Demande U.S. 2001/0053543 citées ci-dessus.

La présente invention sera mieux comprise à l'aide du complément de description qui va suivre, qui se réfère à des exemples non-limitatifs montrant la capacité d'une NDH-II *d'Agrobacterium* à interagir avec la chaîne photosynthétique de transport d'électrons de *Chlamydomonas reinhardtii* et à réduire les plastoquinones, et illustrant la transformation de *Chlamydomonas reinhardtii* par un polynucléotide codant pour ladite NDH-II.

### EXEMPLE 1 : CLONAGE ET EXPRESSION CHEZ E. COLI DE LA NDH-II D'AGROBACTERIUM TUMEFACIENS.

### I- Isolement du gène de la NDH-II

Le gène *Agtundh22* codant pour la NDH-II d'*Agrobacterium tumefaciens* (N° d'accès NCBI : AI2824 ; N° d'accès SWISSPROT : Q8UDU6) a été amplifié de l'ADN génomique *d'Agrobacterium tumefaciens* (souche C58) en utilisant le couple d'amorces suivant :
Sens : N2Ag.mfe.F
   CGCC*AATT*G**ATG**CAAGAACATCATGTT (SEQ ID NO : 5)
Anti-sens : N2Ag.6His.PstR
   AAAA*CTGCAG***TCA**ATGATGATGATGATGATGGGCCTCGTCCTTCAGCG (SEQ ID NO : 6)

Les sites de restriction *mfe*I et *Pst*I (en italique) ont été insérés dans les amorces sens et anti-sens, respectivement en amont et en aval des codons start et stop (en gras). Une étiquette composée de 6 codons histidine (souligné) a été insérée en amont du codon stop. L'amplification a été réalisée dans les conditions suivantes :
- *Mélange réactionnel :*
   Tampon réactionnel spécifique contenant 1,5 mM MgCl₂
   Amorces à 0,5 µM final
   dNTP mix à 200 µM final
   300 ng d'ADN
   1,5 unités d'Expand High Fidelity Taq polymérase (Roche)
- *Conditions d'amplification:*
   3 min 95°C + 1 min à 80°C : 1 cycle
   1 min à 95°C + 1 min à 70°C (-0,5°C à chaque cycle) + 2 min à 72°C : 20 cycles
   1 min à 95°C + 1 min à 60°C + 2 min à 72°C : 10 cycles
   6 minutes à 72°C : 1 cycle

Le produit d'amplification comprend la séquence codant pour la NDH-II, et en 3' de celle-ci, une séquence codant pour une étiquette composée de 6 codons histidine.

### II- Expression chez E.coli et purification

### 1- Clonage

Le produit d'amplification a été digéré par *mfeI* et *PstI* (New England Biolab, protocole recommandé par le fabricant) et introduit par ligation (ligase de New England Biolab, protocole recommandé par le fabricant) dans un vecteur d'expression digéré par *EcoRI* et *PstI.*

Ce vecteur, le pSD80, porte une cassette de résistance à l'ampicilline, un fort promoteur Taq et le gène répresseur Laq iQ (PATEL et DUNN, J. Bacteriol. 177 : 3917-3922,1995 ; SMITH et al., Biochem. 35 : 8805-8814, 1996).

Le produit de ligation a ensuite été introduit par électroporation dans *E. coli* dH10ß.

Les transformants résistants à l'ampicilline ont ensuite été criblés par PCR en utilisant les mêmes amorces et les même conditions que précédemment indiqué, afin de vérifier la présence du gène *Agtundh2.*

La construction a ensuite été vérifiée par séquençage en utilisant des amorces spécifiques du vecteur pSD80 ainsi que des amorces internes au gène *Agtundh2.*

| | |
|---|---|
| PSD80.F | 5'-GAGCTGTTGACAATTAAT-3' (SEQ ID NO : 7) |
| PSD80.R | 5'-AGGACGGGTCACACGCGC-3' (SEQ ID NO: 8) |
| N2Ag.307.F | 5'-TGGCCACCGGCGCGCGT-3' (SEQ ID NO : 9) |
| N2Ag.650.f | 5'-TGCGAAGGAAGCGCTTGA-3' (SEQ ID NO : 10) |
| N2Ag.901.R | 5'-TTCCTGATTGACCGCGG-3' (SEQ ID NO : 11) |

Après avoir vérifié que la séquence et l'insertion étaient correctes, ce plasmide a été nommé pSDN2Ag6H et a servi à co-transformer par électroporation la souche *E. coli* dH10 ß avec un vecteur (pRare, Novagen) portant les 6 plus rares ARNt *d'E. coli.*

Un des co-transformants résistants à l'ampicilline et au chloramphénicol a été choisi pour l'expression et la purification de la protéine NDH-II 6His.

### 2- Expression

Une culture de milieu LB (Luria Berthani), en présence d'ampicilline et de chloramphénicol, d'un volume de 1 L dans un erlenmeyer de 2,5 L a été inoculée au 1/50 à partir d'une préculture de 15h, et incubée à 37°C sous agitation forte, jusqu'à une densité optique de 0,5.

L'expression de la NDH-II 6His a été induite pendant 2h par 0,5 mM d'isopropylthio-ß-D-galactoside (IPTG).

Les cellules ont ensuite été collectées par centrifugation et rincées avec du milieu de culture neuf et conservées à -80°C.

### 3- Purification

Le protocole de purification a été publié par BJÖRKLÖF et al. (FEBS Letts., 467 : 105-110, 2000).

Les cellules ont été décongelées et resuspendues dans 2,5 mM EDTA, 0,2 mM PMSF (phénylméthylsulfonyl fluoride), 200 mM Tris-Cl, pH8, à la concentration d'environ 1 g pour 10 ml. 300 µg/ml de lysozyme ont été ajoutés, et le mélange a été incubé pendant 1 h dans la glace.

La suspension a ensuite été centrifugée pendant 60 min à 120 000 x g. Le culot a été soumis à un choc osmotique par resuspension dans un tampon 10 mM de potassium phosphate pH8, contenant 2 mM EDTA, 0,2 mM PMSF, et homogénéisé à l'aide d'un potter, puis re-centrifugé (60 min, 120 000 x g).

La fraction membranaire a été reprise dans le tampon précédent et homogénéisée avec un potter. Les membranes ont ensuite été solubilisées par addition de dodécylmaltoside (DM), à la concentration finale de 0,2% (poids/vol) et un ratio détergent/protéine de 0,1, et de NaCl à 500 mM de concentration finale. La suspension est agitée et incubée dans la glace pendant 30 min puis centrifugée (60 min, 120 000 x g).

La purification de l'enzyme a été réalisée par chromatographie d'affinité en moyenne pression sur une colonne de nickel (HiTrap chelating column 5 ml, Amersham Biosciences, Uppsala), à 4°C, au moyen d'un système de chromatographie (Åktå FPLC, Amersham Biosciences, Uppsala). La colonne a d'abord été prééquilibrée par du tampon potassium phosphate à 50 mM (pH 7,5) contenant 500 mM de NaCl, 0,2% de DM et 20 mM d'imidazole. Après avoir déposé l'échantillon, la colonne a été rincée par la solution précédente sans imidazole (solution A) pour éliminer toutes les protéines non fixées. La colonne a enfin été éluée par une concentration croissante d'une solution contenant 250 mM d'imidazole (solution B). Des fractions de 5 ml ont été collectées aux différentes proportions de solution concentrée en imidazole (10%, 15%, 50%, 100%).

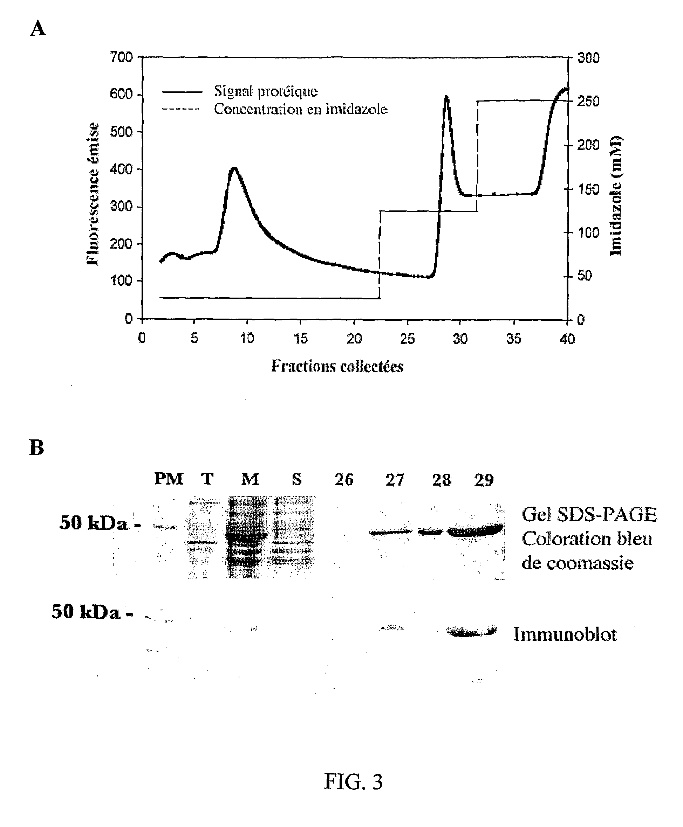

Un pic protéique détecté par fluorescence est apparu sur le chromatogramme pour une concentration de 125 mM d'imidazole (Figure 3A). Le contenu protéique des fractions collectées (26-29) est analysé par éléctrophorèse sur un gel d'acrylamide à 13% en condition dénaturante (SDS-PAGE) (Figure 3B). Pour comparaison, les échantillons correspondants à la fraction totale (T) de départ ainsi qu'aux fractions soluble (S) et membranaire (M), ont été également analysés.

Les protéines ont ensuite été transférées sur membrane de nitrocellulose et marquées par immunoblot avec un anticorps primaire monoclonal (Sigma), dirigé contre une séquence polyhistidine, pendant 1h, puis un anticorps secondaire, dirigé contre les IgG de souris, couplé à la phosphatase alcaline, pendant 1h également.

On observe sur la Figure 3B (29) la présence d'une forte bande marquée par l'anticorps dont le poids moléculaire est inférieur à 50 kDa, ce qui correspond à la taille de la protéine attendue (44 kDa). On retrouve cette protéine dans la fraction membranaire (M) et pas dans la fraction soluble (S).

La fraction 29 a été concentrée par ultrafiltration jusqu'à un volume de 900 µl. Un volume de 220 µl de glycérol pur a été ajouté et l'enzyme a été conservée à -80°C. La concentration de l'enzyme a été évaluée sur un gel SDS-PAGE par comparaison avec une gamme étalon de BSA (Serum Albumine Bovine)

### III-Caractérisation de l'enzyme

### 1- Analyse du coenzyme

La nature du coenzyme a été déterminée par fluorimétrie comme décrit par FANG et BEATTIE (Biochem. 41 : 3065-3072, 2002). L'enzyme purifiée a été portée à ébullition pendant 3 à 4 min puis centrifugée.

Le surnageant a été analysé par HPLC en utilisant une colonne Supelcosil LC-DP de 150 x 4,6 mm (Supelco). La phase mobile est composée à 80% de 0,1 % TFA dans l'eau et à 20% de 0,1% TFA dans l'acétonitrile à 40%. Le débit dans la colonne à température ambiante est de 1 ml/min. Les longueurs d'excitation et d'émission du détecteur de fluorescence ont été réglées respectivement à 450 et 525 nm. Des standards de FAD (Sigma) et FMN (Sigma) ont été analysés en parallèle.

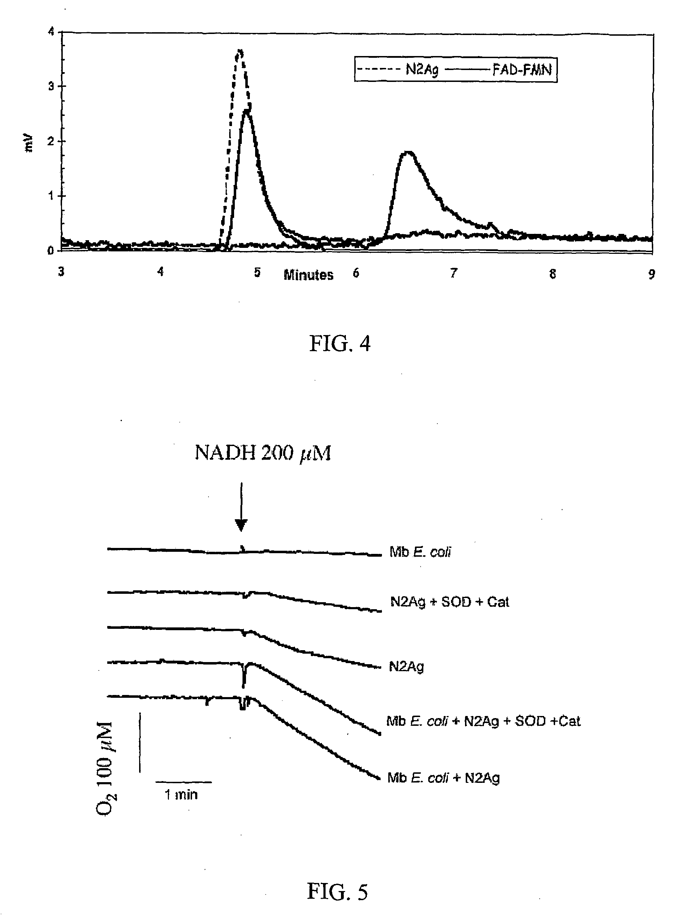

On peut voir sur la Figure 4 que le pic d'émission de l'échantillon d'enzyme (N2Ag) correspond exactement au pic du standard de FAD.

Ces résultats montrent que le cofacteur de la NDH-II *d'Agrobacterium tumefaciens* est le FAD.

### 2- Mesure de l'activité enzymatique sur des membranes bactériennes.

Dans l'expérience suivante, la capacité de cette enzyme à transférer des électrons à la chaîne respiratoire d'*E*. *coli* a été déterminée.

L'activité de la NDH-II d'*Agrobacterium tumefaciens* a été dosée à l'aide de membranes d'une souche d'*E*. *coli* déficiente en NDH-I et NDH-II (*ndh::tet*; *nuoB::nptl*).

La souche *E. coli* ANN0222 (souche mère AN398 ; WALLACE et YOUNG, Biochim. Biophys. Acta. 461 : 84-100, 1977) a été cultivée dans 50 ml de LB-tétracycline jusqu'à la fin de la phase exponentielle de croissance (DO = 1). Les cellules ont ensuite été collectées par centrifugation (15 min, 3200 x g) et lavées deux fois avec 10 ml de solution contenant 200 mM Tris-Cl pH 8, 2.5 mM EDTA et 0.2 mM PMSF. Elles ont ensuite été cassées par deux passages à la presse de French à la pression de 16 000 p.s.i.

La fraction membranaire a été récupérée par centrifugation (30 min, 48500 x g) et resuspendue dans 200 µl de tampon d'analyse (50 mM de tampon phosphate, pH 7,5 et 150 mM NaCl).

La consommation d'O₂ des membranes d'*E*. *coli* ANN0222, est mesurée au moyen d'une électrode de Clark (DW2/2, Hansatech, King's Lynn, England). Après ajout de l'enzyme purifiée, cette méthode a permis de déterminer son activité spécifique.

Le mélange réactionnel contenant 10 µl de membranes bactériennes, 1,5 µl d'enzyme purifiée (conservée à 1 mg/ml) a été préincubé pendant 10 min dans la glace avant d'être dilué dans 990 µl de tampon d'analyse (50 mM de tampon phosphate, pH 7,5 et 150 mM NaCl) et introduit par pipettage dans la chambre d'analyse de l'électrode. La réaction a été suivie à 25°C en présence de concentrations croissantes de NADH ouNADPH comme donneurs d'électrons.

En suivant la réaction en absence de membranes bactériennes, il a été observé que l'enzyme présente une activité de prise directe d'O₂. Celle-ci, fortement réduite en présence de SOD et catalase, est liée à la formation d'espèces activées de l'oxygène (Figure 5).

Afin de mesurer uniquement l'activité oxydase respiratoire liée aux membranes, de la catalase (1000 U/ml) et de la superoxyde dismutase (SOD) (500 U/ml) ont été ajoutées au mélange réactionnel. L'effet d'inhibition du diphénylène iodonium (DPI) a été quantifié après 10 min d'incubation dans la glace en présence du mélange enzyme-membranes. Les cinétiques d'inhibition ont été réalisées en présence de 200 µM de NADH ou de 2 mM de NADPH.

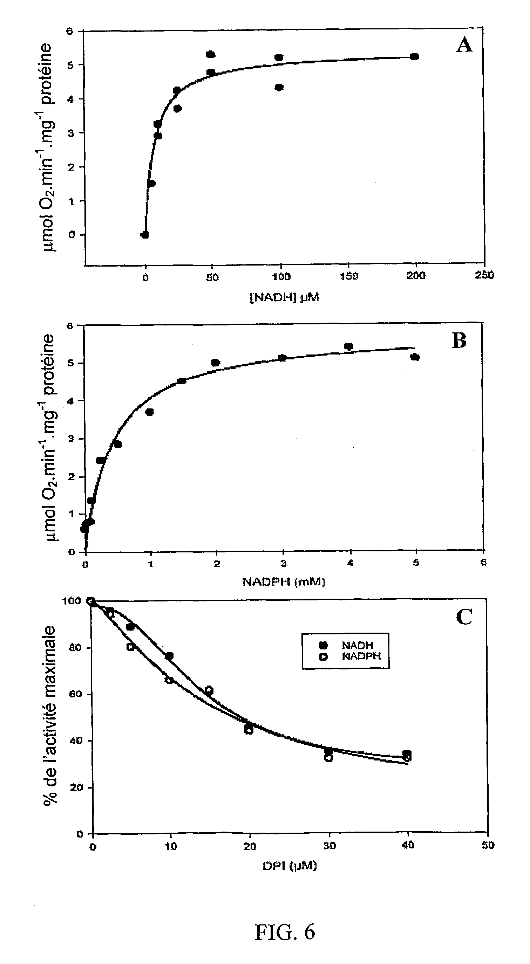

L'activité maximale de la NDH-II *d'Agrobacterium* (Vmax = 5 µmol O₂.min⁻¹.mg⁻¹ protéine) est comprise dans l'ordre de grandeur des autres NDH-II décrites dans la littérature. A pH physiologique, cette enzyme a une forte affinité pour le NADH (Km =10 µM) (Figure 6A), mais également une affinité non négligeable pour le NADPH (Km = 50 µM) (Figure 6B), ce qui est rare pour une enzyme bactérienne. Cette caractéristique est intéressante pour faire fonctionner cette enzyme dans le chloroplaste, car ce compartiment cellulaire contient du NADPH en abondance. L'enzyme est bien affectée par les inhibiteurs classiques des NDH-II, et notamment par le DPI avec un I₅₀ de 10 µM (Figure 6C).

### EXEMPLE 2: MISE EN EVIDENCE DE LA REDUCTION DES PLASTOQUINONES DE CHLAMYDOMONAS REINHARDTII PAR LA NDH-II D'AGROBACTERIUM

L'activité de réduction des plastoquinones par la NDH-II *d'Agrobacterium* a été analysée en mesurant la fluorescence de la chlorophylle. En présence d'un éclairement faible non-actinique, la mesure de la fluorescence de la chlorophylle fournit une indication de l'état redox des plastoquinones.

### I- Préparation des membranes thylakoïdiennes.

Une culture de 200 ml de *Chlamydomonas reinhardtii* (de type sauvage 137c) a été prélevée en phase exponentielle de croissance (environ 5x10⁶ cellules.ml⁻¹), puis centrifugée (5 min, 1000 x g). Les cellules ont été lavées dans du tampon HEPES-NaOH à 35 mM (pH 7,2), resuspendues dans 10 ml de tampon de lyse (50 mM Tricine-NaOH pH 8, 10 mM NaCl, 5 mM MgCl₂, 1% BSA, 1 mM benzamidine et 1mM PMSF) et conservées au froid et à l'obscurité pour les étapes suivantes.

La suspension a été passée deux fois à travers une presse de French à 2000 p.s.i. Le lysat a d'abord été centrifugé (5 min, 4°C, 500 x g) pour éliminer les cellules non lysées, et les membranes de thylacoïdes ont ensuite été culottées à 10 000 x g pendant 10 min et resuspendues dans 250 à 500 µl de tampon d'analyse (50 mM Tricine-NaOH pH 7,2, 10 mM NaCl et 5 mM MgCl₂).

### II- Réductioln des plastoquinones

La fluorescence du photosystème II a été mesurée grâce à un fluorimètre à lumière modulée (PAM 101-103, Walz, Effeltrich, Allemagne).

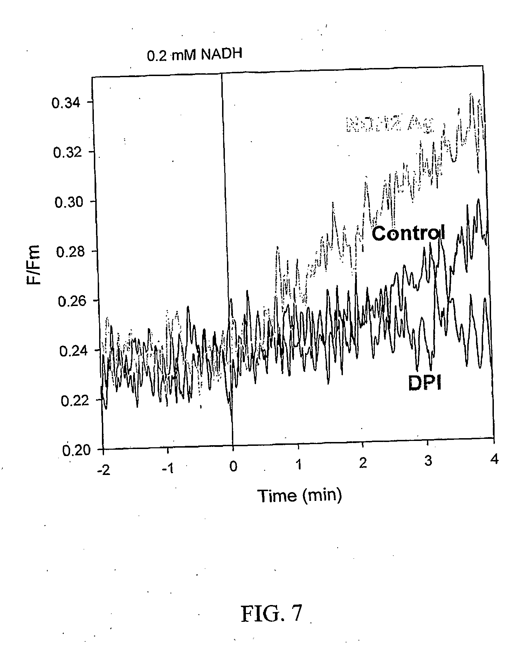

Une lumière modulée non actinique (650 nm, 1,6 kHz) a été utilisée pour déterminer le niveau de fluorescence chlorophyllienne F₀. Le niveau maximal de la fluorescence chlorophyllienne Fₘ a été mesuré sous un flash saturant de 1 seconde (environ 1000 µmol photons.m⁻².s⁻¹). Afin d'éviter la réoxydation des plastoquinones, les expériences ont été réalisées en condition anaérobie par ajout dans le mélange réactionnel de glucose (20 mM), glucose oxydase (2 mg.ml⁻¹) et catalase (1000 unités.ml⁻¹).
Les membranes thylacoïdiennes ont été incubées avec ou sans la NDH-II (1,5 µg.ml⁻¹ de concentration finale) pendant 10 min dans la glace et le taux de réduction des plastoquinones a été comparé avant et après addition de 200 µM de NADH. Les résultats sont présentés sur la Figure 7 (NDH2Ag = présence de la NDH-II d'*Agrobacterium*; Control = présence enzyme native seule ; DPI = effet d'inhibition du DPI)

La Figure 7 montre que la fluorescence de la chlorophylle mesurée sur des membranes thylacoïdiennes de *Chlamydomonas* augmente après ajout de NADH. Cette augmentation correspond à la réduction non photochimique des PQ par l'enzyme native de l'algue. Cependant, en présence de la NDH-II *d'Agrobacterium* purifiée (NHD2Ag), l'augmentation de la fluorescence est plus rapide et plus intense. La réduction des plastoquinones est donc supérieure à celle effectuée par l'enzyme native seule (control). Ceci est la démonstration de la capacité de la NDH-II d'*Agrobacterium* à interagir avec la chaîne photosynthétique de transport d'électrons.

Afin de quantifier cet effet de la NDH-II *d'Agrobacterium* sur le flux d'électrons du NAD(P)H jusqu'aux accepteurs du PSI, des mesures de consommation d'oxygène ont été réalisées en présence de méthyl viologène (un accepteur du PSI),de DCMU (un inhibiteur du PSII) et d'un donneur d'électrons NADH (200 µM) ou NADPH (2 mM).

Les thylacoïdes de *Chlamydomonas* (50 µl, correspondant à 85 µg.ml⁻¹ de chlorophylle) sont placés dans une électrode de Clark contenant 900 µl de tampon d'analyse (50 mM Tricine-NaOH pH 7,2 , 10 mM NaCl et 5 mM MgCl₂, 25 µM DCMU, 50 µM méthyl viologène, SOD (500 U.ml⁻¹), catalase (1000 U.ml⁻¹), 4 µM de myxothiazol, 800 µM de SHAM (acide salicylhydroxamique). Ces deux derniers composés sont ajoutés afin d'inhiber la prise d'O₂ respiratoire.

Dans ces conditions, la fraction de la prise d'oxygène photo-induite sensible au DNP-INT (2-iodo-6-isopropyl-3-méthyl-2',4,4'-trinitrodiphenyl éther ; 10 µM), inhibiteur du cytochrome b₆f, est proportionnelle au transfert d'électrons entre le NAD(P)H et la chaîne photosynthétique.

L'ajout de 1,5 µg de NDH-II *d'Agrobacterium tumefaciens* purifiée stimule ce transfert d'électrons de 20 à 45 nmole.min⁻¹.mg⁻¹ chlorophylle lorsque le NADPH est utilisé comme donneur, et de 21 à 67 nmole.min⁻¹.mg⁻¹ chlorophylle lorsque le NADH est utilisé comme donneur.

Cette expérience permet donc de quantifier la stimulation du transfert d'électrons entre le NADPH (ou le NADH) et les plastoquinones thylacoïdiennes par l'ajout de la NDH-II d'*Agrobacterium tumefaciens.* La stimulation est d'un facteur 2 environ dans le cas du NADPH et d'un facteur 3 environ dans le cas du NADH.

Ces résultats *in vitro* permettent de supposer qu'une expression hétérologue d'une NDH-II chez *Chlamydomonas reinhardtii* (et plus généralement chez toute algue susceptible de produire de l'hydrogène), qu'elle soit chloroplastique, ou nucléaire avec adressage vers le chloroplaste, peut augmenter l'activité plastoquinone réductase intrachloroplastique et la production d'H₂ associée.

### EXEMPLE 3 : TRANSFORMATION DE CHLAMYDOMONAS REINHARDTII PAR LA SEQUENCE CODANT LA NDH-II D'AGROBACTERIUM TUMEFACIENS

### I- Construction du plasmide pSADN2Ag

Le gène *Agtundh2* a été amplifié par PCR à partir du génome d'*A*. *tumefaciens* (souche C58) en utilisant les amorces suivantes :
ndhAgTu.F
   5'-TCCCCCGGGATGCAAGAACATCATGTT-3' (SEQ ID NO: 12)
   (Tm 66,5°C)
   et
ndhAgTu.R
   5'-CCGCAATTGTCAGGCCTCGTCCTTCAG-3' (SEQ ID NO : 13)
   (Tm 69,5°C)
et dans les conditions suivantes :
- *Mélange rédactionnel:*
   Tampon réactionnel spécifique contenant 1,5 mM MgCl₂
   Amorces à 0,5 µM final
   dNTP mélangés à 200 µM final
   300 ng d'ADN
   1,5 unités d'Expand High Fidelity Taq polymérase (Roche)
- *Conditions d'amplification :*
   3 min 95°C + 1 min à 80°C : 1 cycle
   1 min à 95°C + 1 min à 60°C + 2 min à 72°C : 30 cycles
   6 minutes à 72°C : 1 cycle

Le produit d'amplification a été digéré par *Sma*I/*mfe*I.

Le plasmide pGEND2 (FISHER et ROCHAIX, Mol. Genet. Genomics 265 : 888-894, 2001) a été digéré par *Nae*I/*Eco*RI, pour exciser la séquence codant pour la protéine PsaD, à l'exception du peptide d'adressage chloroplastique. Le fragment excisé a été remplacé par le fragment *Sma*I/*mfe*I obtenu à partir du produit d'amplification du gène *N2Ag.*

Le plasmide résultant, dénommé pSADN2Ag, contient donc la séquence codant pour la NDH-II *d'Agrobacterium tumefaciens,* sous contrôle du promoteur du gène *psa*D, et en fusion traductionnelle avec le peptide d'adressage chloroplastique de la protéine PsaD.

### II- Transformation de Chlamydomonas reinhardtii

Un mutant de *Chlamydomonas reinhardtii* déficient en arginosuccinate lyase (CC-2852 arg7 cwl5 mt+; Chlamydomonas Center, Duke University, Durham, USA), et donc auxotrophe pour l'arginine, a été utilisé. Les algues sont mises en culture sur milieu TAP, dans un volume de 200 mL, (HARRIS, The Chlamydomonas sourcebook, Academic Press, San Diego, 1989) supplémenté par 100 mg/l d'arginine, sous agitation, température de 25°C, et lumière continue d'environ 35 µmol photons.m².s⁻¹.

En fin de phase exponentielle (concentration d'environ 10⁷ cellules/ml), les algues sont concentrées par centrifugation et reprises dans du TAP pour obtenir une suspension à 3.10⁸ cellules/ml.

Le plasmide PSADN2Ag est utilisé avec le plasmide p389 (Chlamydomonas Center, Duke University, Durham, USA ; http://www.chlamy.org/strains/plasmids.html) composé d'un fragment d'ADN nucléaire de Chlamydomonas d'une taille de 7,1 kb, incluant le gène *Arg7,* et cloné en BamHI dans le vecteur pBR329, pour co-transformer les algues.

On mélange dans un tube 300 mg de billes de verre de diamètre 0,5 mm (Sigma), 10 (µl de plasmide PSADN2Ag (=1 µg), 2 µl de plasmide p389 (=0,2 µg) et 350 µl de suspension d'algues (=environ 10⁸ cellules). On agite pendant 15 secondes au moyen d'un vortex réglé à la vitesse maximale. On ajoute ensuite 650 µl de TAP, et après homogénéisation, la suspension est utilisée pour inoculer deux boîtes de pétri (450 µl sur chaque boîte) contenant du milieu TAP (agar 15 g/l) sans arginine.

Au bout de 10 jours d'incubation en lumière continue (35 µmol.m².s⁻¹) et à 25°C, les algues sont repiquées sur le même milieu. Après 2 repiquages on récolte les transformants ayant poussé sans arginine.

Les transformants ayant intégré le gène *N2Ag* sont sélectionnés en détectant la présence de ce gène par PCR.

Les colonies sont lysées selon le protocole suivant :
Chaque colonie est reprise dans 100 µl d'eau stérile (filtration milliQ®). Dans un tube de 1,5 ml on mélange 5 µl de tampon 10X de PCR, 1 µl de SDS à 0.01%, 1 µl de DTT à 200 mM, 3 µl de cellules et 39µl d'H₂O.

On effectue 5 cycles de congélation/décongélation (entre azote liquide et bain-marie à 55°C)

On ajoute 2 µl de protéinase K à 10 µg/ml et on incube 1 h à 55°C.

La protéinase K est inactivée par un traitement de 5 minutes à 95°C.

La PCR est effectuée en utilisant les amorces ndhAgTu.F et ndhAgTu.R, et dans les conditions suivantes :
- *Mélange réactionnel :*
   Pour 25 µl de volume final
   2,5 µl de tampon 10X
   2 µl de dNTP mix
   1,25 µl de chaque amorce
   2,5 µl de BSA 10X
   3 µl de lysat de cellules
   0,3 µl de polymérase Taq (Qiagen)
   12,2 µl H20
- *Conditions d'amplification :*
   3 min 95°C + 1 min à 80°C : 1 cycle
   1 min à 95°C + 1 min à 60°C + 2 min à 72°C : 30 cycles
   6 minutes à 72°C : 1 cycle

Un contrôle positif a été effectué en remplaçant l'ADN génomique de *Chlamydomonas* par le plasmide ayant servi à la transformation.

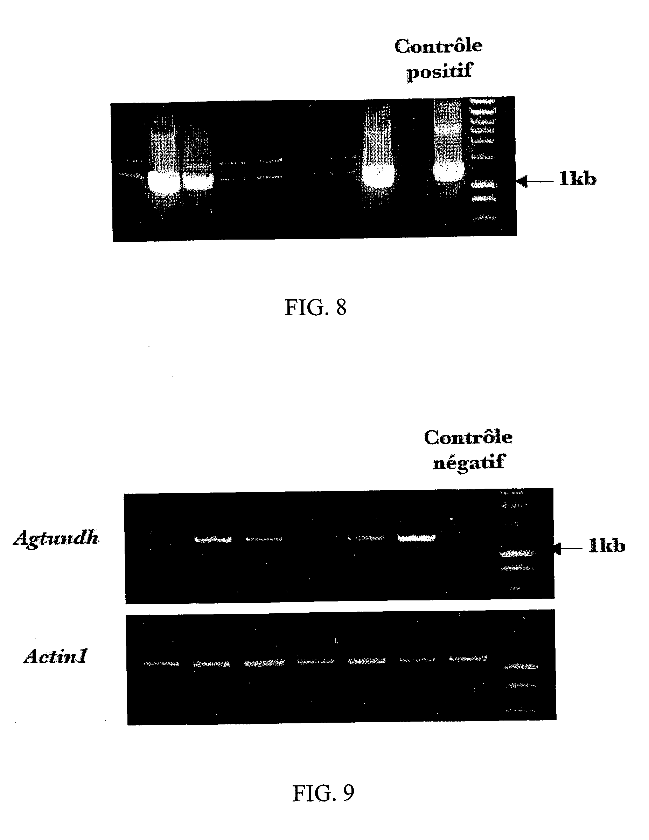

Les produits d'amplification ont été analysés par migration sur un gel d'agarose à 1% et visualisés après marquage du gel au bromure d'éthydium (Figure 8). Un marqueur de poids moléculaire a été déposé en parallèle sur le gel afin d'estimer la taille des produits d'amplification obtenus.

Les transformants ayant intégré le gène de la NDH-II présentent une bande d'environ 1,2 kb également observée sur le témoin positif. La proportion de co-transformants (= ayant intégré les deux plasmides) est d'environ 50%.

### III- Expression de la NDH-II :

L'expression du gène de la NDH-II d'*Agrobacterium* a été vérifiée par RT-PCR dans 6 co-transformants. Les ARN totaux sont extraits à partir de 25ml de culture en phase exponentielle avec le kit RNeasy®, selon les indications du fabricant (Qiagen). Les ADNc sont préparés à l'aide du kit Omniscript®, selon le protocole préconisé par le fabricant (Qiagen) en ajustant le volume des différents ARNs pour obtenir 2µg dans chaque réaction.

La PCR est effectuée en utilisant les amorces ndhAgTu.F et ndhAgTu.R, et dans les conditions suivantes :
- *Mélange réactionnel :*
   Tampon réactionnel spécifique contenant 1,5 mM MgCl₂
   Amorces à 0,5 µM final
   dNTP mélangés à 200 µM final
   300 ng d'ADNc
   1,5 unités d'Expand High Fidelity Taq polymerase (Roche)
- *Conditions d'amplification :*
   3 min 95°C + 1 min à 80°C : 1 cycle
   1 min à 95°C + 1 min à 60°C + 2 min à 72°C : 30 cycles
   6 minutes à 72°C : 1 cycle

Un contrôle négatif a été réalisé pour vérifier que les amplicons observés ne proviennent pas d'une amplification de l'ADN nucléaire persistant après l'étape de digestion incluse dans le protocole d'extraction des ARNs. Pour ce contrôle, les 300 ng d'ADNc obtenus après l'étape de transcriptase réverse sont remplacés par 5 µl de la solution d'ARN précédant cette étape.

Pour contrôler l'efficacité du protocole d'extraction ainsi que le niveau d'expression de la NDH-II, l'ADNc de l'Actine 1 qui est une protéine exprimée constitutivement et en quantité importante, a été amplifié en parallèle.

Les produits d'amplification ont été analysés par migration sur un gel d'agarose à 1% et visualisés après marquage du gel au bromure d'éthydium (Figure 9). Un marqueur de poids moléculaire a été déposé en parallèle sur le gel afin d'estimer la taille des produits d'amplification obtenus.

Les co-transformants exprimant le gène de la NDH-II présentent une bande d'environ 1,2 kb correspondant à la taille de l'insert. Le contrôle négatif ne présente aucune bande, ce qui signifie que les préparations d'ARN n'étaient pas contaminées par de l'ADN génomique, alors qu'il présente bien un produit d'amplification pour l'actine. Tous les co-transformants testés expriment le gène de la NDH-II à des niveaux différents mais du même ordre de grandeur que celui de l'actine.

### EXEMPLE 4 : COMPLEMENTATION FONCTIONNELLE D'UNE SOUCHE D'E. COLI DEFICIENTE EN NDH

### I - Complémentation-test de croissance

La fonctionnalité de la protéine Agtundh2 *in vivo* dans un hôte hétérologue, a été testée en déterminant sa capacité à restaurer la croissance d'une souche mutante d'E. *coli,* ANN.0222, déficiente en NDH-1 et en NDH-2. Cette souche pousse normalement sur milieu LB medium, mais est incapable de pousser sur milieu minimal M9 supplémenté avec du mannitol comme unique source de carbone.

La souche *E. coli* ANN.0222 a été transformée chimiquement (CaCl₂) avec le plasmide pSDN2Ag6H. Les transformants ont été sélectionnés sur milieu LB agar (supplémenté avec 1% tryptone, 0.5% d'extrait de levure et 0.5% NaCl, pH 7) contenant de l'ampicilline (100µg/ml). Les transformants et la souche d'origine ont été cultivés jusqu'au milieu de la phase exponentielle à 37°c dans un milieu LB liquide contenant de l'ampicilline (100µg/ml). Les cellules ont été rincées avec du milieu M9 stérile supplémenté avec du mannitol comme seule source de carbone (1X sels M9, 2x10⁻³ M MgSO₄, 10⁻⁴ M CaCl₂, 0.4% mannitol). Les cellules ont ensuite été diluées dans du milieu M9/mannitol et inoculées sur un milieu solide M9/mannitol/agar contenant de l'ampicilline et différentes concentrations d'IPTG. Les boîtes de Pétri ont été incubées à 37°c pendant 2 jours. Comme contrôle, les mêmes cellules ont été inoculées en parallèle sur des boîtes contenant du milieu LB agar contenant de l'ampicilline et diverses concentrations d'IPTG, puis incubées à 37°C durant la nuit.

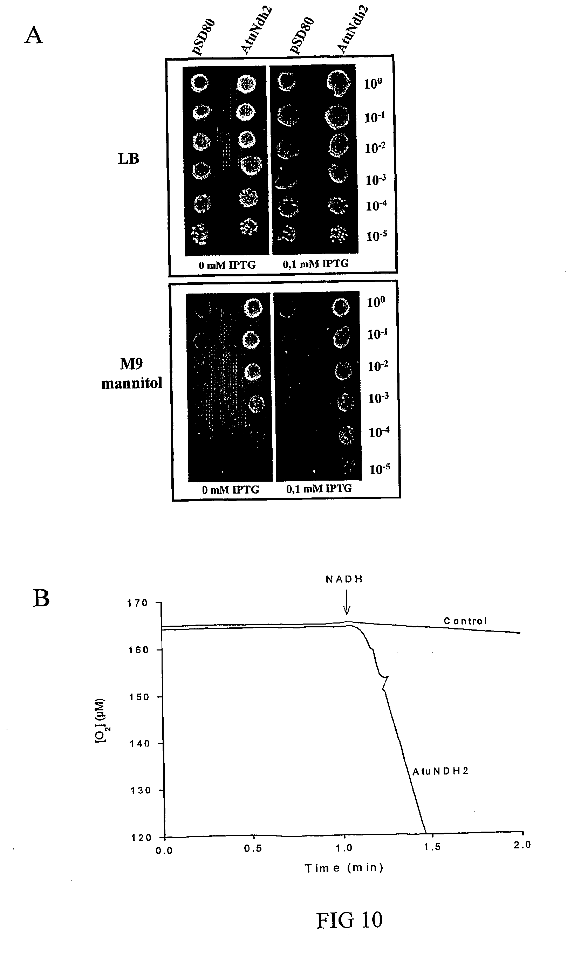

Les résultats sont illustrés par la Figure 10A, qui représente la formation de colonies sur milieu riche (LB) et sur milieu minimal supplémenté avec du mannitol (M9+mannitol) pour la souche ANN0222 et le transformant qui exprime Agtundh2 (noté AtuNdh2 sur la Figure). Les concentrations d'IPTG sont indiquées au-dessus de chaque piste correspondante.

La croissance des souches mutantes non transformées est considérablement limitée sur milieu minimal, que ce soit en absence ou en présence d'IPTG (0.1 mM). Par contre, la croissance sur milieu minimal de la souche exprimant Agtundh2 a été restaurée après induction par 0.1 mM IPTG. Une complémentation partielle du mutant a été observée même en l'absence d'IPTG, ce qui suggère un certain niveau d'expression IPTG-indépendant de la protéine.

### II - Activité NADH déshydrogénase membranaire

Deux lots de fractions membranaires de *E*. *coli* ANN.0222 ont été préparés, à partir d'une culture de la souche témoin sur milieu LB et d'une culture de la souche ayant incorporé le plasmide pSDN2Ag6H cultivée sur milieu LB en présence de 0.1 mM IPTG. Les cultures ont été menées jusqu'à une densité optique de 1 à 600 nm. Les cellules ont été collectées par centrifugation (15 min, 3200 x g), rincées deux fois et resuspendues dans10 ml de tampon A (200 mM Tris-Cl pH 8, 2.5 mM EDTA et 0.2 mM PMSF; (30)). Les cellules ont ensuite été cassées par deux passages à travers une presse de French à 16 000 p.s.i. La fraction membranaire, collectée par centrifugation (30 min, 4°C, 48500 x g), a été reprise dans 200 µl de tampon B (50 mM tampon phosphate, pH 7.5 et 150 mM NaCl). La prise d'O₂ a été mesurée à l'aide d'une électrode de Clark (DW2/2, Hansatech, King's Lynn, England), sur des aliquotes de 2 µl de ces fractions membranaires, dilués dans 1 ml de tampon A, à 25°C et en présence de NADH.

Les résultats sont illustrés par la Figure 10B, qui représente la prise d'O₂ en présence de NADH de membranes d'*E*. *coli* ANN0222 préparées à partir de la souche de référence (control) et de la souche exprimant Agtundh2.

Alors qu'aucune activité de prise d'O₂ n'est détectée dans les membranes de la souche témoin en réponse à l'addition de NADH, une prise d'O₂ importante est en revanche détectée dans les mêmes conditions dans les membranes de la souche transformée par pSDN2Ag6H. (Fig. 10B). Exprimée dans des membranes d'*E*. *coli,* Agtundh2 est capable d'oxyder NADH et NADPH avec des vitesses maximales du même ordre, mais avec une affinité bien plus forte pour le NADH.

### III - Expression de la protéine

L'expression de la protéine Agtundh2 dans la souche ANN.0222 transformée a été confirmée par immunodétection.

Un sérum de lapin a été produit contre la protéine Agtundh2 purifiée (Agro-Bio, Villeny, France). Les fractions protéiques soluble et membranaire ont été extraites comme indiqué ci-dessus. Ces fractions protéiques, ainsi que des aliquotes de la protéine Agtundh2 purifiée, ont été chargées sur gel 10% SDS-PAGE et soumises à une électrophorèse, puis transférées sur membranes de nitrocellulose. Les membranes de nitrocellulose ont ensuite été incubées 30 minutes dans du lait (3% de lait écrémé en poudre dans de l'eau additionnée de 0.1% TBST), puis rincées avec du 0.1% TBST incubées à nouveau durant 1h30 avec l'anticorps anti-Agtundh2 dilué au 1/10000^{ème}. Les membranes ont ensuite été rincées trois fois pendant 10 minutes avec du TBST 0.1% puis incubées pendant 1 h avec un anticorps secondaire anti-lapin conjugué à la phosphatase alcaline, dilué au 1/10000^{ème}. La réaction de détection a été réalisée suivant le protocole recommandé par le fabricant (Sigma).

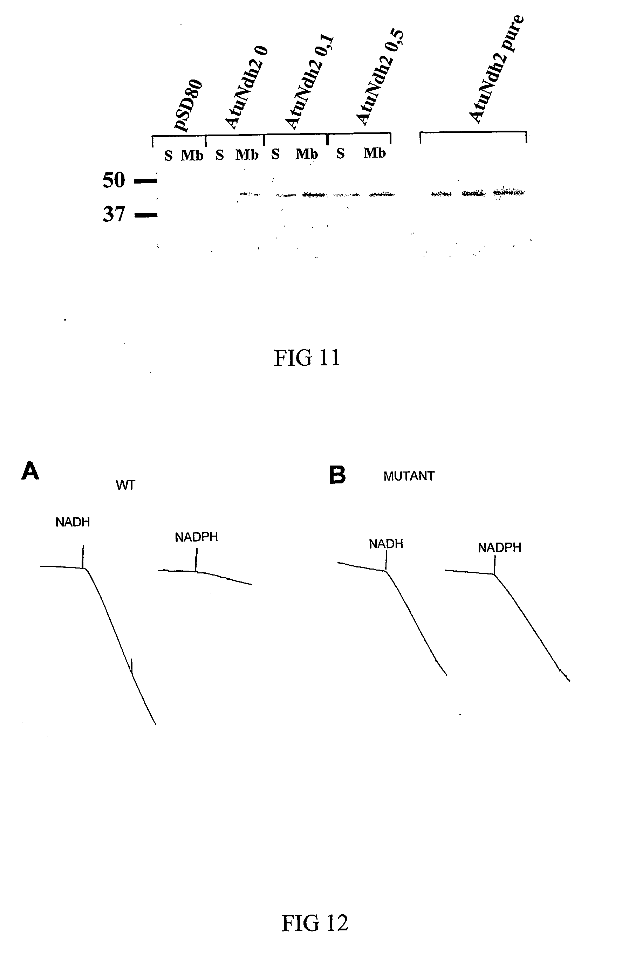

Les résultats sont illustrés par la Figure 11 : Panneau de gauche: immunodétection sur fractions protéiques soluble (S) et membranaire (Mb) de la souche *E. coli* ANN0222, transformée soit avec le vecteur pSD80 "vide", soit avec la construction portant *Agtundh2*, et exposée à 0, 0.1 or 0.5 mM d'IPTG. La charge du gel correspondait *in fine* à une dilution au 1/400^{ème} des fractions soluble et membranaire de 50 ml de culture récoltés à DO₆₀₀=1 (soit 2.5 µg de protéines sur les pistes correspondant aux fractions membranaires et 30 µg de protéines sur celles correspondant aux fractions solubles). Panneau de droite: immunodétection de Agtundh2 purifiée, à 0.1, 0.2 et 0.3 µg/piste de gauche à droite.

La plus grande part de la protéine a été trouvée dans les fractions membranaires, une proportion mineure étant détectée dans les protéines solubles. Une expression significative d'Agtundh2 a été détectée même en l'absence d'IPTG, ce qui est en accord avec la complémentation partielle observée dans ces conditions (cf. Fig. 10A).

Pour déterminer l'efficacité catalytique de Agtundh2 exprimée dans *E. coli*, l'activité spécifique a été estimée à partir des quantités déterminées par Western blot.

Sur la base des intensités relatives des signaux obtenus pour la protéine Agtundh2 purifiée et pour les fractions membranaires, la quantité de protéine Agtundh2 dans les expériences illustrées par la Fig. 10B a été estimée à 0.6 µg protéine. Les prises d'O₂ maximales dans ces conditions sont ainsi estimées à 13.2/0.6 = 22 nmol O₂ min⁻¹. µg⁻¹ protein, soit 44 nmol NADH min⁻¹. µg⁻¹. protéine.

### EXEMPLE 5: MODIFICATION DE LA SPECIFICITE RELATIVE D'AGTUNDH2 POUR LE NADH ET LE NADPH

### I - Amplification du gène Agtundh2

Le gène *Agtundh2* a été amplifié à partir du plasmide pSDN2Ag6H en utilisant le couple d'amorces suivant :
*sens : N2Ag .NcoI
   ***ATG**GAAGAACATCATGTTGTCGTC* (SEQ ID NO : 14)

Le site de restriction NcoI (en italique) a été inséré dans l'amorce de manière à chevaucher le codon start du gène (en gras) et en modifiant la quatrième base de la séquence (C devient G).
*Anti-sens : N2Ag.XbaI
CCG*TCTAGA***TCA**GGCCTCGTCCTTCAGCGT (SEQ ID NO : 15)

Le site de restriction XbaI (en italique) a été inséré dans l'amorce anti-sens en aval du codon stop du gène (en gras).

L'amplification a été réalisée dans les conditions suivantes:
*1-Mélange réactionnel:*
   Tampon réactionnel spécifique 1X
   1,5 µM MgSo4 final
   Amorces à 0,5 µM final
   dNTP mix à 200 µM final
   200 ng d'ADN
   1 unité de Pfx platinum (Invitrogen)
*2-Conditions d'amplication :*
   2 min 94°C : 1 cycle
   30 sec à 94°C +1 min à 55°C + 3 min à 68°C : 25 cycles
   10 min à 68°C : 1 cycle

### II - Introduction de la mutation E201Q dans la séquence du gène Agtundh2 :

La mutation du gène *Agtundh2* E201Q a été introduite au sein de la séquence du gène par la technique de mutagénèse dirigée dite « PCR fusion ». Les fragments correspondant à la première étape d'amplification ont été obtenus en utilisant le plasmide pSDN2Ag6H comme matrice. Les couples d'amorces utilisés sont les suivants :
* Sens : N2Ag .NcoI ;
*Anti-sens : N2Ag.E201Q.R
   AGGGCCGGC**CTG**CACAAGCAA (SEQ ID NO : 16)

L'amorce N2Ag.E201Q.R permet d'introduire la mutation E201Q (en gras).

Ces deux amorces permettent d'obtenir le fragment 5' contenant la mutation E201Q.
* Anti-sens : N2Ag.XbaI ;
* Sens : N2Ag.E201 Q.F
   TTGCTTGTGCAGGCCGGCCCT (SEQ ID NO : 17)

L'amorce N2Ag.E201Q.F permet d'introduire la mutation E201Q (en gras).

Ces deux amorces permettent d'obtenir le fragment 3' contenant la mutation E201Q.
- *Mélange réactionnel :*
   Tampon réactionnel spécifique 1X
   1,5 µM MgSo4 final
   Amorces à 0,5 µM final
   dNTP mix à 200 µM final
   200 ng d'ADN
   1 unité de Pfx platinum (Invitrogen)
- *Conditions d'amplification :*
   2 min 94°C : 1 cycle
   30 sec à 94°C +1 min à 55°C + 3 min à 68°C : 25 cycles
   10 min à 68°C : 1 cycle

Les deux fragments d'amplification renferment la mutation d'intérêt introduite grâce aux amorces N2Ag.E201 Q.F et N2Ag.E201 Q.R.

La seconde étape d'amplification est réalisée en mélangeant les deux fragments d'amplification obtenus lors de l'étape précédente. Ces deux fragments vont s'hybrider grâce à leur séquence commune (TTGCTTGTGCAGGCCGGCCCT) (SEQ ID NO : 17) et vont ainsi constituer la matrice. L'amplification est réalisée en utilisant les deux amorces N2Ag.NcoI et N2Ag.XbaI, dans les conditions suivantes :
- *Mélange rédactionnel :*
   Tampon réactionnel spécifique 1X
   1,5 µM MgSo4 final
   Amorces à 0,5 µM final
   dNTP mix à 200 µM final
   200 ng de fragment N2Ag.NcoI/N2Ag.E201Q.R
   200 ng de fragment N2Ag.XbaI/N2Ag.E201Q.F
   1 unité de Pfx platinum (Invitrogen)
- *Conditions d'amplification :*
   2 min 94°C : 1 cycle
   30 sec à 94°C +1 min à 55°C + 6 min à 68°C : 30 cycles
   10 min à 68°C : 1 cycle

### III - Clonage des constructions

Le produit d'amplification a été digéré par Ncol et XbaI (New England Biolabs, protocole recommandé par le fournisseur) et introduit par ligation (T4 DNA ligase de New England Biolabs, protocole recommandé par le fournisseur) dans le vecteur pBAD24 digéré par NcoI et XbaI. Ce vecteur porte une cassette de résistance à l'ampicilline, un promoteur de type pBAD inductible à l'arabinose. Les produits d'amplification ont ensuite été introduits par électroporation dans *E. coli* dH10β. Les transformants résistants à l'ampicilline ont été sélectionnés et la présence de l'insert a été vérifiée par extraction de l'ADN plasmidique et digestion par NcoI et HindIII.

Les deux constructions ont ensuite été vérifiées par séquençage en utilisant des amorces internes au gène *Agtundh2* (N2Ag.E201Q.F; N2Ag.NcoI ; N2Ag.E201 Q.R ; N2Ag.XbaI).

Le séquençage a montré que la mutation désirée (E201 Q) a bien été introduite. Le plasmide a été nommé E201Qc1.

### IV - Activité de la protéine modifiée

L'affinité de la protéine purifiée pour le NADH et le NADPH a été déterminée.

Les résultats sont illustrés sur la Figure 12: Alors que la protéine de type sauvage (A) présente une activité nettement supérieure à 2001 µM NADH par rapport à celle enregistrée à 200 µM NADPH, la protéine modifiée (B) présente une affinité comparable pour les deux substrats.

### EXEMPLE 6: IDENTIFICATION D'UNE NDH2 DE CHLAMYDOMONAS REINHARDTII A LOCALISATION CHLOROPLASTIQUE

### I - Amplification du gène

L'ARN total a été isolé de cultures de *C. reinhardtii* sur milieu TAP (prélevées entre 1x10⁶ et 1x10⁷ cellules/ml), en utilisant le QIAGEN RNeasy® Plant Mini Kit, conformément aux instructions du fabricant. La synthèse de cDNA a été réalisée en utilisant l'Omniscript^{™} Reverse Transcriptase system (QIAGEN) avec une amorce Oligo-dT primer. Les produits de la réaction ont été amplifiés par PCR avec la polymérase *turbo pfu* (Stratagene) dans un OmniGene Hybrid thermocycler.
- *Conditions d'amplification de l'ADNc:*
   Dénaturation à 94°C: 5 min
   30 s à 94°C, 40 s à 72°C et 2 min à 72°C: 5 cycles
   30 s à 94°C, 40 s à 68°C et 2 min à 72°C: 5 cycles
   30 s à 94°C, 40 s à 65°C et 2 min à 72°C: 5 cycles
   30 s à 94°C, 40 s à 62°C et 2 min à 72°C: 15 cycles
   Extension à 72°C: 5 min.

Les amorces utilisées pour l'amplification de N2Cr2 sont :
5'-ATGCATAGCCTTGATGGCCAAAAC-3' et (SEQ ID NO : 18)
5'-TCACACTCGCGAGATGTCGCG-3'. (SEQ ID NO : 19)

Ces deux amorces ont permis d'amplifier un ADNc (1662 pb) par RT-PCR. La nouvelle séquence ainsi identifiée a été baptisée *N2Cr2.* L'ADNc correspondant à *N2Cr2* code pour un polypeptide de 533 acides aminés avec une masse prédite de 60,5 kDa.

En comparant la séquence de cet ADNc avec les séquences génomiques disponibles de *C. reinhardtii*, il a été constaté qu'il correspond en fait à une protéine tronquée de 65 acides aminés à son extrémité N-tenninale.

La séquence complète d'ADNc de *N2Cr2* est représentée dans la liste de séquences en annexe sous le numéro SEQ ID NO: 3, et la séquence polypeptidique déduite sous le numéro SEQ ID NO: 4. L'ADNc cloné correspond aux nucléotides 196-1857 de la séquence SEQ ID NO: 3, et code pour un polypeptide correspondant aux acides aminés 67-619 de la séquence SEQ ID NO: 4.

La protéine codée par la séquence d'ADNc de 1662 pb clonée, bien que tronquée à son extrémité N-terminale, code pour une protéine présentant effectivement une activité NADH déshydrogénase, et qui a permis de générer un anticorps reconnaissant la protéine N2Cr2 chez *Chlamydomonas*, comme illustré ci-après.

### II - Clonage du gène dans pSD80

La région codante *N2Cr2* de la séquence d'ADNc de 1662 pb a été amplifiée par PCR en utilisant des amorces correspondant aux parties N- et C-terminales de la séquence étendues de bases additionnelles conférant les sites de restriction *EcoRI, SmaI* et une séquence codante 6-Histidine. Les sites *EcoRI* et *SmaI* (soulignés) ont été insérés dans les amorces forward (F) reverse (R), respectivement en amont et aval des codons start et stop. La séquence codante 6-Histidine (en italique), été insérée dans l'amorce F en aval du codon start de la région codante *N2Cr2.* Les séquences des oligonucléotides sont donc:
*F-EcoRI:
   5'-CGGAATTCATG*CATCATCATCATCATCAT*CATAGCCTTGATGGCCAAAAC-3' (SEQ ID NO : 20) et
*R-SmaI:
   5'-TCCCCCGGGTCACACTCGCGAGATGTCGCG-3'(SEQ ID NO : 21).

L'ADNc amplifié a été digéré par *EcoRI* et *SmaI* et inséré dans le vecteur d'expression pSD80 portant la résistance à la carbénicilline (Patel and Dunn, 1995) préalablement digéré par *EcoRI* et *SmaI*. Le plasmide résultant, nommé pSD80-*N2Cr2*, a été vérifié par séquençage puis utilisé pour transformer la souche *E. coli* DH10β par électroporation. Les cellules portant pSD80-N2Cr2 sont nommées DH10β(pSD80-N2Cr2). L'expression de N2Cr2 étiquetée 6-His a été réalisée dans 2 litres de milieu LB inoculé avec 10 mL d'une culture sur la nuit de DH10β(pSD80-N2Cr2), puis placé (140 rpm) à 37°C en présence de carbénicilline (50 µg.mL⁻¹). Environ 4 h après inoculation, les cellules ayant atteint une DO₆₀₀ₙₘ de 0,5, l'expression de N2Cr2 étiquetée 6-His a été initiée par addition de 100 µM isopropylthio-β-D-galactoside (IPTG). Les cellules ont été récoltées 5 h après induction, rincées avec 25 mL de milieu LB (centrifucation à 4 355 g, 1 min, 4°C) et stockées à -80°C.

### III - Purification sur colonne de la protéine étiquetée

L'isolement et la purification par affinité pour le Nickel de N2Cr2-6His ont été réalisés selon le protocole de BJÖRKLÖF et al. (2000, précité). Les cellules DH10β(pSD80-N2Cr2) ont été décongelées sur glace et reprises dans un tampon 2.5 mM EDTA, 0.2 mM PMSF, 200 mM Tris-Cl, pH 8.0 à environ 1 g pour 10 mL. Du lysozyme a été ajouté et le mélange agité durant 1 h sur glace. Après cela, les cellules on été cassées par deux passages successifs à la presse de French (16 000 psi). Le lysat a été centrifugé à 12 000 g pendant 1 h, puis le surnageant a été collecté et utilise pour la purification de N2Cr2-6His par chromatographie d'affinité pour le nickel, en utilisant une résine Histrap HP (Amersham Bioscience). La colonne a été pré-équilibrée avec un tampon contenant 20 mM triethanolamine, 500 mM NaCl, 25 mM imidazole, pH=7.5 (tampon A). Après avoir chargé l'échantillon, la colonne a été rincée deux fois avec deux volumes de colonne de tampon A, puis rincée avec un volume similaire de 50 mM Tris-Cl, 0,2% (w/v) dodecyl maltoside, pH=7.5 (tampon B) et enfin avec un volume de colonne de 50 mM Tris-Cl, 0,2% (w/v) dodecyl maltoside, 10 mM CaCl₂, pH=7.5 (tampon C). Après ce "lavage calcium", la colonne a été rincée à nouveau avec trois volumes de tampon B et deux volumes de tampon A. N2Cr2-6His a ensuite été éluée en utilisant un gradient d'imidazole réalisé à partir de 20 mM triéthanolamine, 500 mM NaCl, 300 mM imidazole, pH=7.5. N2Cr2-6His a été décrochée de la colonne à environ 180 mM imidazole; la fraction correspondante a été concentrée par ultrafiltration en utilisant le système "Amicon Ultra 30 kDa" (Millipore). Du glycerol a été ajouté pour une concentration finale de 50% (v/v), et l'enzyme stockée à -80°C.

### IV - Production d'un anticorps contre la protéine recombinante :

En utilisant le protocole de purification ci-dessus, 2 mg de protéine ont été obtenus. La pureté de la protéine a été vérifiée sur gel coloré au bleu de Coomassie et le matériel a été utilisé pour produire un sérum dirigé contre N2Cr2, par immunisation d'un lapin.

### V - Fractionnement cellulaire:

### Obtention des fractions totales, mitochondriales et solubles :

Afin de déterminer la localisation subcellulaire de la protéine au sein des cellules de *Chlamydomonas reinhardtii* nous avons procédé à un fractionnement cellulaire nous permettant de séparer la fraction débris, mitochondries et protéines solubles. Nous avons pour cela utilisé une souche sans parois (CW15) permettant d'obtenir un fractionnement cellulaire "doux" par lyse à la presse de Yeda.

400ml d'une culture de CW15 en phase exponentielle de croissance de *Chlamydomonas reinhardtii* sont centrifugés 5 min à 500g à 4°C; puis le culot est repris dans 50 ml de HEPES 35mM pH7,2 et recentrifugé 5 min à 500g à 4°C. Le culot repris dans 12,5 ml de tampon de lyse (Tricine-NaOH 50mM, NaCl 10mM, MgCl₂ 5mM, pH=8) est introduit dans une presse de Yeda pour effectuer le fractionnement. Les cellules sont incubées sous N₂ dans la presse durant 6 min à 8 bars, puis récupérées goutte à goutte.

L'échantillon est ensuite centrifugé 5 min à 500g à 4°C. Le culot est collecté et repris dans 2 ml de SDS 1%. Le surnageant est centrifugé à 3220g durant 8 min à 4°C. Le culot contient des thylacoïdes, qui ne sont pas récupérés car contaminés en membranes mitochondriales. Le surnageant est centrifugé 1 heure à 100 000g à 4°C. Le culot contient les mitochondries et est repris par 1ml de SDS 1%. Le surnageant constitue la fraction soluble. Les trois fractions sont précipitées à l'acétone (80% pour les protéines totales et membranaires et 60% pour la fraction soluble). Un échantillon de chaque fraction est dosé en utilisant la technique à l'acide bicinchoninique (BC Assay kit, Uptima UP40840A, INTERCHIM).

### Extraction des thylacoïdes

Afin d'obtenir des fractions chloroplastiques non contaminées par des membranes mitochondriales, une purification sur gradient de percoll (COURNAC et al., J. Biol. Chem., 275, 23, 17256-17262, 2000) a été effectuée.

Pour cela, 600ml d'une culture de Cw15 en milieu de phase exponentielle de croissance sont centrifugés à 500g durant 5 min à 4°C. Le culot est lavé une fois dans 50 ml de HEPES 35mM pH=7,2 et recentrifugé 5 min à 500g à 4°C. Le culot est repris dans 12,5 ml de tampon A (0,3 M sorbitol, 50 mM HEPES-KOH, pH8,2, 2 mM EDTA, 5mM MgCl₂) et introduit dans la presse de Yeda pour 3 min à 4,5 bars. Le matériel est déposé au dessus d'un gradient de Percoll (40-60%) et centrifugé durant 20' à 4000g. L'anneau correspondant aux chloroplastes intacts est collecté et dilué dans 10 fois son volume de tampon A. L'échantillon est centrifugé 15 min à 3220g et le culot est repris dans 3,5 ml de SDS 0,2% puis précipité à 80% d'acétone.

L'échantillon est dosé selon la méthode à l'acide bicinchoninique.

### VI - Western blotting :

100 µg de chaque fraction sont préparés en centrifugeant le volume adéquat 10 min à 10 000 rpm et en resuspendant le culot dans du tampon de charge 1X. 5 µl (soit 5 µg) de chaque fraction sont chargés sur un gel SDS-PAGE 10%. En contrôle environ 0,1 µg de protéine purifiée sont également chargés sur le gel. Après migration les protéines sont transférées (transfert semi-sec)sur une membrane de nitrocellulose (Life sciences, BioTrace NT).

Les protéines sont ensuite marquées par immunoblot en utilisant pour anticorps primaire l'anticorps obtenu contre la protéine recombinante (AGRO-BIO). L'incubation dure 1 heure à température ambiante. Puis l'anticorps secondaire (anti IgG de lapin) couplé à un fluorochrome est ajouté pour une heure. La détection est réalisée en utilisant le scanner infrarouge Odyssey de la compagnie LICOR.

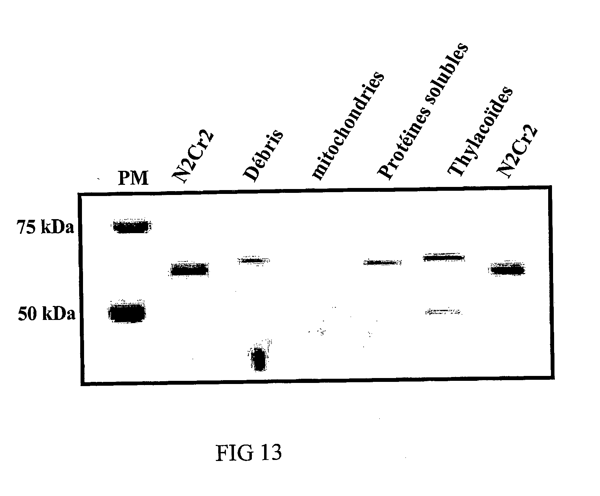

Les résultats sont illustrés par la Figure 13

La bande réagissant de la manière la plus intense est localisée dans la fraction thylacoïdienne. Elle est de taille légèrement plus importante que la N2Cr2 recombinante produite. Cette différence de taille est probablement due au fait que la protéine recombinante est tronquée en N-ter par rapport à la séquence prédite de la protéine mature chez *Chlamydomonas.*

### EXEMPLE 7 : CLONAGE D'AGTUNDH2 DANS LE PLASMIDE PXX6 POUR TRANSFORMATION CHEZ CHLAMYDOMONAS REINHARDTII

### I - Construction du fragment à cloner :

Afin de pouvoir exprimer la protéine Agtundh2 chez *Chlamydomonas reinhardtii* un produit de fusion entre le gène *rbcS2* de *Chlamydomonas reinhardtii* et le gène *Agtundh2* de *Agrobacterium tumefasciens* a été créé. Pour cela la séquence du peptide transit de *rbcS2* a été placée en amont du codon start de *Agtundh2* et la séquence de la région 3' UTR de *rbcS2* a été placée en aval du codon stop de *Agtundh2*, en utilisant la technique de PCR fusion.

### Amplification du peptide de transit de rbcS2

Dans un premier temps le peptide transit de *rbcS2* a été amplifié à partir d'ADN génomique de *Chlamydomonas reinhardtii* en utilisant le couple d'amorces suivant :
*N2Ag.XhoI :
   CGG*CTCGA*G**ATG**GCCGCCGTCATTGCCAAG (SEQ ID NO : 22)

Le site de restriction de l'enzyme XhoI (en italique) a été introduit en amont du codon start (en gras) du gène *rbcS2.*
*TP.ndh.R :
   GACGACAACATGATGTTCTTGCAT*CTGGTTGGCCTGAGCCGGGGCAGC* (SEQ ID NO : 23)

La région correspondant à la séquence du peptide de transit de *rbcS2* est indiquée en italique, et celle correspondant à l'extension *Agtundh2* en caractères normaux.

### -MELANGE REACTIONNEL :

Tampon réactionnel spécifique 1X
1,5 µM MgSo4 final
Amorces à 0,5 µM final
dNTP mix à 200 µM final
200 ng d'ADN
1 unité de Pfx platinum (Invitrogen)

### -CONDITIONS D'AMPLIFICATION:

2 min 94°C : 1 cycle
30 sec à 94°C +1 min à 60°C + 3 min à 68°C : 30 cycles
10 min à 68°C : 1 cycle

### Amplification du gène Agtundh2

Le gène *Agtundh2* a été amplifié à partir du plasmide pSDN2Ag6H en utilisant le couple d'amorces suivant :
* TP.ndh.F :
   *GCTGCCCCGGCTCAGGCCAACCAG***ATG**CAAGAACATCATGTTGTCGTC (SEQ ID NO : 24)
   Une extension correspondant à la fin de la séquence du peptide transit de *rbcS2* (en italique) a été introduite en amont du codon start (en gras) de *Agtundh2.*
* rbcS2.3'.R :
   *GCTCAGATCAACGAGCGCCTCCAT***TCA**GGCCTCGTCCTTCAGCGTCTC (SEQ ID NO : 25)

Une extension correspondant au début de la séquence de la région 3' UTR de *rbcS2* (en italique) a été introduite en aval du codon stop de *Agtundh2* (en gras).
- *Mélange réactionnel :*
   Tampon réactionnel spécifique 1X
   1,5 µM MgSo4 final
   Amorces à 0,5 µM final
   dNTP mix à 200 µM final
   200 ng d'ADN
   1 unité de Pfx platinum (Invitrogen)
- *Conditions d'amplification:*
   2 min 94°C : 1 cycle
   30 sec à 94°C +1 min à 55°C + 3 min à 68°C : 30 cycles
   10 min à 68°C : 1 cycle

### Amplification de la région 3' UTR de rbcS2 :

La région 3' UTR de rbcS2 a été amplifiée à partir de l'ADN génomique de *Chlamydomonas reinhardtii* en utilisant le couple d'amorces suivant :
rbcS2.3'.F
   *GAGACGCTGAAGGACGAGGCCTGA*ATGGAGGCGCTCGTTGATCTGAGC (SEQ ID NO : 26)
N2Ag.KpnI :
   *CGGGGTAC*CCTGCAAATGCTGTCTCCA (SEQ ID NO : 27)

Pour l'amorce rbcS2.3'.F, une extension correspondant à la région C-terminale de *Agtundh2* (en italique) a été introduite en amont du début de la séquence 3' UTR de *rbcS2* (en caractères normaux).

Pour l'amorce N2Ag.KpnI, le site de restriction de l'enzyme KpnI (en italique) a été introduit en aval de la fin de la séquence de la région 3' UTR de *rbcs2* (en caractères normaux).
- *Mélange réactionnel :*
   Tampon réactionnel spécifique 1X
   1,5 µM MgSo4 final
   Amorces à 0,5 µM final
   dNTP mix à 200 µM final
   200 ng d'ADN
   1 unité de Pfx platinum (Invitrogen)
- *Conditions d'amplification :*
   2 min 94°C : 1 cycle
   30 sec à 94°C +1 min à 60°C + 3 min à 68°C : 30 cycles
   10 min à 68°C : 1 cycle

### Amplification d'un fragment de fusion entre le peptide transit de rbcs2 et le gène Agtundh2.

Les fragments d'amplification correspondant au peptide transit de rbcS2 et au gène *Agtundh2* sont mélangés et servent de matrice pour l'amplification du fragment de fusion en utilisant le couple d'amorces suivant (voir ci-dessus) :
N2Ag.XhoI
rbcS2.3'.R
   - *Mélange réactionnel:*
      Tampon réactionnel spécifique 1X
      1,5 µM MgSo4 final
      Amorces à 0,5 µM final
      dNTP mix à 200 µM final
      200 ng de fragment peptide transit de rbcs 2
      200ng de fragment du gène *Agtundh2*
      1 unité de Pfx platinum (Invitrogen)
   - *Conditions d'amplification :*
      2 min 94°C : 1 cycle
      30 sec à 94°C +1 min à 55°C + 6 min à 68°C : 30 cycles
      10 min à 68°C : 1 cycle

### Amplification du fragment de fusion entre le peptide transit de rbcS2, le gène Agtundh2 et la région 3' UTR de rbcS2 :

Le fragment de fusion obtenu précédemment (Peptide de transit de rbcS2 + gène *Agtundh2*) est mélangé au fragment correspondant à la région 3' UTR de rbcS2 pour servir de matrice à la dernière étape d'amplification en utilisant le couple d'amorces suivant : N2Ag.XhoI ; N2Ag.KpnI.
- *Mélange réactionnel :*
   Tampon réactionnel spécifique 1X
   1,5 µM MgSo4 final
   Amorces à 0,5 µM final
   dNTP mix à 200 µM final
   200 ng de fragment peptide de transit + gène *Agtundh2*
   200ng de fragment 3' UTR de rbcS2
   1 unité de Pfx platinum (Invitrogen)
- *Conditions d'amplification :*
   2 min 94°C : 1 cycle
   30 sec à 94°C +1 min à 55°C + 6 min à 68°C : 30 cycles
   10 min à 68°C : 1 cycle

### II - Clonage du fragment de fusion dans le plasmide pXX6 :

Le produit de fusion a été digéré par *XhoI* et *KpnI* (New England Biolabs, protocole recommandé par le fournisseur) et introduit par ligation (T4 DNA ligase New England Biolabs, protocole recommandé par le fournisseur) dans le plasmide pXX6 préalablement digéré par *XhoI* et *KpnI*.

Le plasmide pXX6 est décrit par (FUHRMANN M et al., Plant Mol. Biol., 55, 6, 869-881, 2004). Il porte une cassette de résistance à l'ampicilline, une région du promoteur fort Hsp de *Chlamydomonas reinhardtii* optimisant la transcription , du gène placé en aval, le promoteur constitutif du gène *rbcS2* de *Chlamydomonas reinhardtii*, le premier intron du gène *rbcS2* de *Chlamydomonas reinhardtii*.

Le produit de ligation a ensuite été introduit par électroporation dans *E. coli* dH10β.

Les transformants résistants à l'ampicilline ont été sélectionnés et la présence de l'insert a été vérifiée par extraction de l'ADN plasmidique et digestion par les enzymes XhoI et KpnI.

La construction a ensuite été vérifiée par séquençage en utilisant les amorces suivantes : N2Ag.XhoI ; N2Ag.KpnI ; TP.ndh.R ; TP.ndh.F ; rbcS2.3'.F.

Après avoir vérifié que la séquence et l'insertion étaient correctes, ce plasmide, nommé pXX6N2Ag est utilisé pour co-transformer par la technique des billes de verre la souche CW15 de *Chlamydomonas* avec le plasmide aphVIII portant la résistance à la paromomycine et la souche 388 (auxotrophe pour l'arginine) de *Chlamydomonas* avec le plasmide pArg portant le gène Arg7 codant pour l'arginosuccinate lyase.

## Revendications

1. Procédé pour accroître la capacité d'une algue verte à produire de l'hydrogène, **caractérisé en ce qu'**il comprend la transformation de ladite algue par un polynucléotide codant pour une NAD(P)H déshydrogénase de type II (NDH-II), et l'expression de ladite NDH-II dans ladite algue, ladite NDH-II :
- présentant au moins un exemplaire du motif consensus GxGxxG où « G » représente une glycine et « x » représente un acide aminé quelconque,
- ayant la capacité à catalyser la réduction des quinones des chaînes de transfert d'électrons par l'oxydation du NADH ou du NADPH, en utilisant un cofacteur flavinique, et
- étant, dans sa forme active, un monomère de 30 à 60 kDa ou un homodimère.

2. Procédé selon la revendication 1, **caractérisé en ce que** ladite NDH-II est une NDH-II mutante obtenue à partir d'une NDH-II utilisant préférentiellement le NADH, par substitution du résidu glutamate ou aspartate situé à la fin du second feuillet beta du motif beta-alpha-beta de liaison des pyridine nucléotides, par un résidu polaire neutre.

3. Procédé selon une quelconque des revendications 1 ou 2, **caractérisée en ce que** ladite NDH-II est choisie parmi :
- la NDH-II Agtundh2 *d'Agrobacterium tumefaciens*, définie par la séquence SEQ ID NO : 2, ou une NDH-II mutante obtenue à partir d'Agtundh2 par substitution du résidu glutamate en position 201 de la séquence SEQ ID NO: 2 par un résidu glutamine ;
- la NDH-II N2Cr2 de *Chlamydomonas reinhardtii*, définie par la séquence SEQ ID NO: 4, ou par un fragment de celle-ci comprenant les acides aminés 67-619 de la séquence SEQ ID NO: 4, ou une NDH-II mutante obtenue à partir de N2Cr2 par substitution du résidu glutamate en position 285 de la séquence SEQ ID NO: 4 par un résidu glutamine.

4. Algue verte transformée par un polynucléotide codant pour une NDH-II telle que définie dans une quelconque des revendications 1 à 3.

5. Utilisation d'une algue verte selon la revendication 4 pour la production d'hydrogène.

## Patentansprüche

1. Verfahren zur Verstärkung der Fähigkeit einer Grünalge, Wasserstoff zu produzieren, **dadurch gekennzeichnet, dass** es die Transformation der genannten Alge durch ein Polynucleotid, welches eine NAD(P)H-Dehydrogenase des Typs II (NDH-II) codiert, und Expression der NDH-II in der Alge umfasst, wobei die NDH-II:
- wenigstens ein Exemplar des Consensus-Motivs GxGxxG aufweist, worin "G" ein Glycin darstellt und "x" eine beliebige Aminosäure darstellt,
- die Fähigkeit hat, die Reduktion der Chinone der Elektronentransportketten durch Oxidation des NADH oder des NADPH zu katalysieren, wobei ein Flavin-Cofaktor verwendet wird, und
- in ihrer aktiven Form ein Monomer mit 30 bis 60 kDa oder ein Homodimer ist.

2. Verfahren gemäß Anspruch 1, **dadurch gekennzeichnet, dass** die NDH-II eine NDH-II-Mutante ist, die ausgehend von einer NDH-II, die vorzugsweise NADH nutzt, durch Substitution des Glutamat- oder Aspartatrestes, der sich am Ende des zweiten beta-Faltblatts des Bindungsmotivs beta-alpha-beta der Pyridinnucleotide befindet, durch einen neutralen polaren Rest, erhalten wurde.

3. Verfahren gemäß einem der Ansprüche 1 oder 2, **dadurch gekennzeichnet, dass** die NDH-II ausgewählt ist aus:
- der NDH-II Agtundh2 von *Agrobacterium tumefaciens*, definiert durch die Sequenz SEQ ID NO: 2, und einer NDH-II-Mutante, erhalten ausgehend von Agtundh2 durch Substitution des Glutamatrestes in Position 201 der Sequenz SEQ ID NO: 2 durch einen Glutaminrest;
- der NDH-II N2Cr2 von *Chlamydomonas reinhardtii,* definiert durch die Sequenz SEQ ID NO: 4 oder durch ein Fragment dieser, das die Aminosäuren 67 bis 619 der Sequenz SEQ ID NO: 4 umfasst, oder einer NDH-II-Mutante, erhalten ausgehend von N2Cr2 durch Ersatz des Glutamatrestes in Position 285 der Sequenz SEQ ID NO: 4 durch einen Glutaminrest.

4. Grünalge, transformiert durch ein Polynucleotid, das für eine NDH-II, wie sie in einem der Ansprüche 1 bis 3 definiert ist, codiert.

5. Verwendung einer Grünalge gemäß Anspruch 4 zur Herstellung von Wasserstoff.

## Claims

1. Method for increasing the capacity of a green alga to produce hydrogen, **characterised in that** it comprises transforming said alga with a polynucleotide coding for a type II NAD(P)H dehydrogenase (NDH-II) and expressing said NDH-II in said alga, said NDH-II:
- having at least one copy of the consensus motif GxGxxG, wherein "G" represents a glycine and "x" represents any amino acid,
- having the ability to catalyse the reduction of quinones of electron transport chains by oxidation of NADH or NADPH, using a flavin cofactor, and
- being, in its active form, a monomer of from 30 to 60 kDa or a homodimer.

2. Method according to claim 1, **characterised in that** said NDH-II is a mutant NDH-II obtained from an NDH-II using preferably NADH, by substitution of the glutamate or aspartate residue situated at the end of the second beta sheet of the pyridine-nucleotide-binding beta-alpha-beta motif with a neutral polar residue.

3. Method according to either claim 1 or claim 2, **characterised in that** said NDH-II is chosen from:
- the NDH-II Agtundh2 from *Agrobacterium tumefaciens*, defined by sequence SEQ ID NO: 2, or a mutant NDH-II obtained from Agtundh2 by substitution of the glutamate residue at position 201 of sequence SEQ ID NO: 2 with a glutamine residue;
- the NDH-II N2Cr2 from *Chlamydomonas reinhardtii,* defined by sequence SEQ ID NO: 4, or by a fragment thereof comprising amino acids 67-619 of sequence SEQ ID NO: 4, or a mutant NDH-II obtained from N2Cr2 by substitution of the glutamate residue at position 285 of sequence SEQ ID NO: 4 with a glutamine residue.

4. Green alga transformed by a polynucleotide coding for a NDH-II as defined in any one of claims 1 to 3.

5. Use of a green alga according to claim 4 for the production of hydrogen.


### SEQUENCE LISTING

<110> COMMISSARIAT A L'ENERGIE ATOMIQUE (CEA)
   BERNARD, Laetitia
   MUS, Florence
   CUINE, Stéphan
   COURNAC, Laurent
   PELTIER, Gilles
   SIMON-DESPLATS, Carine
<120> PRODUCTION D'HYDROGENE PAR EXPRESSION HETEROLOGUE D'UNE NAD(P)H DESHYDROGENASE DE TYPE II CHEZ CHLAMYDOMONAS
<130> MJPbv263-117
<150> FR 04 10715
   <151> 2004-10-11
<160> 27
<170> PatentIn version 3.3
<210> 1
   <211> 1266
   <212> DNA
   <213> Agrobacterium tumefaciens
<220>
   <221> CDS
   <222> (1)..(1266)
<400> 1
   atg caa gaa cat cat gtt gtc gtc gtg ggt gga ggc ttc ggc ggt tta      48
   Met Gln Glu His His Val Val Val Val Gly Gly Gly Phe Gly Gly Leu
   1               5                   10                  15
   caa ctg gtg cat ggg ctg gag ggg gcc cct gtc cgc att aca tta atc      96
   Gln Leu Val His Gly Leu Glu Gly Ala Pro Val Arg Ile Thr Leu Ile
               20                  25                  30
   gac cgt cgc aat cac cac ctg ttt cag ccc ctg ctg tat cag gtt gcg     144
   Asp Arg Arg Asn His His Leu Phe Gln Pro Leu Leu Tyr Gln Val Ala
           35                  40                  45
   acg acg gcg ctg gcg aca tcc gag atc gcc tgg ccg atc cgc cat ctt     192
   Thr Thr Ala Leu Ala Thr Ser Glu Ile Ala Trp Pro Ile Arg His Leu
       50                  55                  60
   tac cgc gac cgc aag gaa gtg acg acc ctg ctt gcg gag gtg acc ggc     240
   Tyr Arg Asp Arg Lys Glu Val Thr Thr Leu Leu Ala Glu Val Thr Gly
   65                  70                  75                  80
   gtg gac cgg gcg gcg cgg acc gtg cag ctg aat tcg ggc cag gtg atc     288
   Val Asp Arg Ala Ala Arg Thr Val Gln Leu Asn Ser Gly Gln Val Ile
                   85                  90                  95
   ggc ttc gat acg ctg gtg ctg gcc acc ggc gcg cgt cac gcc tat ttc     336
   Gly Phe Asp Thr Leu Val Leu Ala Thr Gly Ala Arg His Ala Tyr Phe
               100                 105                 110
   ggt cac gac gaa tgg gag cgt tcc gcc ccc ggc ctc aag acg ctg gaa      384
   Gly His Asp Glu Trp Glu Arg Ser Ala Pro Gly Leu Lys Thr Leu Glu
           115                 120                 125
   gat gca acg aca atc cgc cgc cgc ctg ctt ctg gcc ttc gaa agg gcc      432
   Asp Ala Thr Thr Ile Arg Arg Arg Leu Leu Leu Ala Phe Glu Arg Ala
       130                 135                 140
   gaa ctt gcc acc agt gaa gag gag cgg cag gcg ctg ctg acc ttc gtc      480
   Glu Leu Ala Thr Ser Glu Glu Glu Arg Gln Ala Leu Leu Thr Phe Val
   145                 150                 155                 160
   atc atc ggg gcc ggc ccg acc ggc gtg gaa atg gcg ggc atg atc gcc      528
   Ile Ile Gly Ala Gly Pro Thr Gly Val Glu Met Ala Gly Met Ile Ala
                   165                 170                 175
   gag ctc gcc cac agg gcg ctg ccg gcg gaa ttc cgc aat gtc gat acc      576
   Glu Leu Ala His Arg Ala Leu Pro Ala Glu Phe Arg Asn Val Asp Thr
               180                 185                 190
   aga aag acc cgc gta ttg ctt gtg gag gcc ggc cct cgc gtc ctg ccg      624
   Arg Lys Thr Arg Val Leu Leu Val Glu Ala Gly Pro Arg Val Leu Pro
           195                 200                 205
   gtc ttc acg gag gat ctt tcg acc tat gcg aag gaa gcg ctt gag aag      672
   Val Phe Thr Glu Asp Leu Ser Thr Tyr Ala Lys Glu Ala Leu Glu Lys
       210                 215                 220
   ctc ggc gtc gag gtt ctg ctc gga acg ccg gtg acg gcc tgc acg gat      720
   Leu Gly Val Glu Val Leu Leu Gly Thr Pro Val Thr Ala Cys Thr Asp
   225                 230                 235                 240
   gag ggc gtg acg gtg ggc gag acc tat tat ccc tgc cgc acc gtc gtc      768
   Glu Gly Val Thr Val Gly Glu Thr Tyr Tyr Pro Cys Arg Thr Val Val
                   245                 250                 255
   tgg gcc gcc ggt gtg cag gcc tct ccg gca gcg aag tgg ctg aat gcg      816
   Trp Ala Ala Gly Val Gln Ala Ser Pro Ala Ala Lys Trp Leu Asn Ala
               260                 265                 270
   gcc ggc gac agg gca ggg cgt gtc atc gtc ggg ccg cag ctt cat ctc      864
   Ala Gly Asp Arg Ala Gly Arg Val Ile Val Gly Pro Gln Leu His Leu
           275                 280                 285
   gaa gac gat gct gat atc ttc gtc atc ggc gat acc gcc gcg gtc aat      912
   Glu Asp Asp Ala Asp Ile Phe Val Ile Gly Asp Thr Ala Ala Val Asn
       290                 295                 300
   cag gaa aac ggc aag ccg gtg ccg ggc atc gcg ccc gcg gca aag cag      960
   Gln Glu Asn Gly Lys Pro Val Pro Gly Ile Ala Pro Ala Ala Lys Gln
   305                 310                 315                 320
   cag ggc gct tat gtg gca aag gtc atc aag gcg cga ctg gag ggc aag     1008
   Gln Gly Ala Tyr Val Ala Lys Val Ile Lys Ala Arg Leu Glu Gly Lys
                   325                 330                 335
   ccg ata ccg gca ccc ttc cgc tac agc cat cag ggc aat ctc gcc acc     1056
   Pro Ile Pro Ala Pro Phe Arg Tyr Ser His Gln Gly Asn Leu Ala Thr
               340                 345                 350
   atc ggc aaa cgc gcg gcg gtg atc gat ttc ggc cgg ttc aag ctg aag      1104
   Ile Gly Lys Arg Ala Ala Val Ile Asp Phe Gly Arg Phe Lys Leu Lys
           355                 360                 365
   ggc gtg ctg gca tgg tgg ata tgg ggg ctt gcc cat atc tac ttc ctg      1152
   Gly Val Leu Ala Trp Trp Ile Trp Gly Leu Ala His Ile Tyr Phe Leu
       370                 375                 380
   atc gga acg cgg tcg cgg ctg gcc gtg gcg tgg agc tgg ctg tgg att      1200
   Ile Gly Thr Arg Ser Arg Leu Ala Val Ala Trp Ser Trp Leu Trp Ile
   385                 390                 395                 400
   tat ctg agc ggc cag cac agt gcg cgc ctg att acc cag aaa gag acg      1248
   Tyr Leu Ser Gly Gln His Ser Ala Arg Leu Ile Thr Gln Lys Glu Thr
                   405                 410                 415
   ctg aag gac gag gcc tga                                              1266
   Leu Lys Asp Glu Ala
               420
<210> 2
   <211> 421
   <212> PRT
   <213> Agrobacterium tumefaciens
<400> 2
   Met Gln Glu His His Val Val Val Val Gly Gly Gly Phe Gly Gly Leu
   1               5                   10                  15
   Gln Leu Val His Gly Leu Glu Gly Ala Pro Val Arg Ile Thr Leu Ile
               20                  25                  30
   Asp Arg Arg Asn His His Leu Phe Gln Pro Leu Leu Tyr Gln Val Ala
           35                  40                  45
   Thr Thr Ala Leu Ala Thr Ser Glu Ile Ala Trp Pro Ile Arg His Leu
       50                  55                  60
   Tyr Arg Asp Arg Lys Glu Val Thr Thr Leu Leu Ala Glu Val Thr Gly
   65                  70                  75                  80
   Val Asp Arg Ala Ala Arg Thr Val Gln Leu Asn Ser Gly Gln Val Ile
                   85                  90                  95
   Gly Phe Asp Thr Leu Val Leu Ala Thr Gly Ala Arg His Ala Tyr Phe
               100                 105                 110
   Gly His Asp Glu Trp Glu Arg Ser Ala Pro Gly Leu Lys Thr Leu Glu
           115                 120                 125
   Asp Ala Thr Thr Ile Arg Arg Arg Leu Leu Leu Ala Phe Glu Arg Ala
       130                 135                 140
   Glu Leu Ala Thr Ser Glu Glu Glu Arg Gln Ala Leu Leu Thr Phe Val
   145                 150                 155                 160
   Ile Ile Gly Ala Gly Pro Thr Gly Val Glu Met Ala Gly Met Ile Ala
                   165                 170                 175
   Glu Leu Ala His Arg Ala Leu Pro Ala Glu Phe Arg Asn Val Asp Thr
               180                 185                 190
   Arg Lys Thr Arg Val Leu Leu Val Glu Ala Gly Pro Arg Val Leu Pro
           195                 200                 205
   Val Phe Thr Glu Asp Leu Ser Thr Tyr Ala Lys Glu Ala Leu Glu Lys
       210                 215                 220
   Leu Gly Val Glu Val Leu Leu Gly Thr Pro Val Thr Ala Cys Thr Asp
   225                 230                 235                 240
   Glu Gly Val Thr Val Gly Glu Thr Tyr Tyr Pro Cys Arg Thr Val Val
                   245                 250                 255
   Trp Ala Ala Gly Val Gln Ala Ser Pro Ala Ala Lys Trp Leu Asn Ala
               260                 265                 270
   Ala Gly Asp Arg Ala Gly Arg Val Ile Val Gly Pro Gln Leu His Leu
           275                 280                 285
   Glu Asp Asp Ala Asp Ile Phe Val Ile Gly Asp Thr Ala Ala Val Asn
       290                 295                 300
   Gln Glu Asn Gly Lys Pro Val Pro Gly Ile Ala Pro Ala Ala Lys Gln
   305                 310                 315                 320
   Gln Gly Ala Tyr Val Ala Lys Val Ile Lys Ala Arg Leu Glu Gly Lys
                   325                 330                 335
   Pro Ile Pro Ala Pro Phe Arg Tyr Ser His Gln Gly Asn Leu Ala Thr
               340                 345                 350
   Ile Gly Lys Arg Ala Ala Val Ile Asp Phe Gly Arg Phe Lys Leu Lys
           355                 360                 365
   Gly Val Leu Ala Trp Trp Ile Trp Gly Leu Ala His Ile Tyr Phe Leu
       370                 375                 380
   Ile Gly Thr Arg Ser Arg Leu Ala Val Ala Trp Ser Trp Leu Trp Ile
   385                 390                 395                 400
   Tyr Leu Ser Gly Gln His Ser Ala Arg Leu Ile Thr Gln Lys Glu Thr
                   405                 410                 415
   Leu Lys Asp Glu Ala
               420
<210> 3
   <211> 1857
   <212> DNA
   <213> Chlamydomonas reinhardtii
<220>
   <221> CDS
   <222> (1)..(1857)
<400> 3
   atg aac atg ttg ctt caa caa cag aag ctc gct gcg ggc tgc aag cag        48
   Met Asn Met Leu Leu Gln Gln Gln Lys Leu Ala Ala Gly Cys Lys Gln
   1               5                   10                  15
   cgc agc gtt gcg cag ccc tcg cgc gga tgc gtc gct gcc cac acc ggc        96
   Arg Ser Val Ala Gln Pro Ser Arg Gly Cys Val Ala Ala His Thr Gly
               20                  25                  30
   ctg cgc tct ggg cgc gtt gct tcc cgt caa cga tca gtg acg act gcg       144
   Leu Arg Ser Gly Arg Val Ala Ser Arg Gln Arg Ser Val Thr Thr Ala
           35                  40                  45
   gtc atg aca ccg ccg gcg aag tcg gag tct tcg agc ccg gta tat acc       192
   Val Met Thr Pro Pro Ala Lys Ser Glu Ser Ser Ser Pro Val Tyr Thr
       50                  55                  60
   acc atg agc ctt gat ggc caa aac ctc aag act gct aag cct cgc ctg       240
   Thr Met Ser Leu Asp Gly Gln Asn Leu Lys Thr Ala Lys Pro Arg Leu
   65                  70                  75                  80
   gtt gtg ctg ggt tcc ggc tgg ggc gcc atg tcg ttc ctg aag gcg ctg       288
   Val Val Leu Gly Ser Gly Trp Gly Ala Met Ser Phe Leu Lys Ala Leu
                           85                  90                  95
   ccg acc agc atc agc tcg acc tat gag ctc atc gtc gtc tcg cct cgc      336
   Pro Thr Ser Ile Ser Ser Thr Tyr Glu Leu Ile Val Val Ser Pro Arg
               100                 105                 110
   aac tac ttc ctg tac acg ccg ctg ctg ccg gct gtg gcg acc gga acc      384
   Asn Tyr Phe Leu Tyr Thr Pro Leu Leu Pro Ala Val Ala Thr Gly Thr
           115                 120                 125
   atg gag gag cgt tcc att gtg gag ccc gtg cgc aac ttc atc gtg ggc      432
   Met Glu Glu Arg Ser Ile Val Glu Pro Val Arg Asn Phe Ile Val Gly
       130                 135                 140
   aag ggc gag ttc tac gag gct ctg tgc aag gac att gac ccg gtc gcc      480
   Lys Gly Glu Phe Tyr Glu Ala Leu Cys Lys Asp Ile Asp Pro Val Ala
   145                 150                 155                 160
   aag gag ctg gtg tgc tgc ttc ccg gag gac gcg ggg ctg gac agc gcc      528
   Lys Glu Leu Val Cys Cys Phe Pro Glu Asp Ala Gly Leu Asp Ser Ala
                   165                 170                 175
   tgc ttc aag atg agc tac gac gtg ctg gtc atg gcg gtc ggt tca gtg      576
   Cys Phe Lys Met Ser Tyr Asp Val Leu Val Met Ala Val Gly Ser Val
               180                 185                 190
   aac aac acg ttt ggc atc aag gga gtg gac cag tac tgc ttc tac ttc      624
   Asn Asn Thr Phe Gly Ile Lys Gly Val Asp Gln Tyr Cys Phe Tyr Phe
           195                 200                 205
   aag tcc att gag gac gcc aac agg ctg cgc tcg cgc gtg tcg gag tgc      672
   Lys Ser Ile Glu Asp Ala Asn Arg Leu Arg Ser Arg Val Ser Glu Cys
       210                 215                 220
   ttt gag cgc gcc gcc ctg cct gcc aca ccc gag gag gag cgc aag aag      720
   Phe Glu Arg Ala Ala Leu Pro Ala Thr Pro Glu Glu Glu Arg Lys Lys
   225                 230                 235                 240
   ctg ctg acg ttt gtg gtg gtg ggc ggc ggc ccc acg ggc gtg gag gtg      768
   Leu Leu Thr Phe Val Val Val Gly Gly Gly Pro Thr Gly Val Glu Val
                   245                 250                 255
   gcg gcg gag ctg tac gac atg atc gag gag gac ctc tcc aag ctc tac      816
   Ala Ala Glu Leu Tyr Asp Met Ile Glu Glu Asp Leu Ser Lys Leu Tyr
               260                 265                 270
   ccc aac ctg gtc aag gac gtg tcc atc cag gtg gtg gag ctg atg gac      864
   Pro Asn Leu Val Lys Asp Val Ser Ile Gln Val Val Glu Leu Met Asp
           275                 280                 285
   cac gtg ctg tcc acc tac gac cgc gcc att agc ctg tac acc gcc gag      912
   His Val Leu Ser Thr Tyr Asp Arg Ala Ile Ser Leu Tyr Thr Ala Glu
       290                 295                 300
   cag ttc aag cgc gcg ggc atc aag ctg gtc ctc aac agt cgt gtg gcg      960
   Gln Phe Lys Arg Ala Gly Ile Lys Leu Val Leu Asn Ser Arg Val Ala
   305                 310                 315                 320
   tcc gtg gag gac ggc gtg gtg cgg gtg gtg aac aag gcc aac gag agc      1008
   Ser Val Glu Asp Gly Val Val Arg Val Val Asn Lys Ala Asn Glu Ser
                   325                 330                 335
   gtc gac atc aag ttt ggc gcg tgt gtg tgg gcc act ggc atc gcc atg      1056
   Val Asp Ile Lys Phe Gly Ala Cys Val Trp Ala Thr Gly Ile Ala Met
               340                 345                 350
   aac ccg ctg gtg cgg cag ctg cag gag aag ctg ccg ggg cag agc cac      1104
   Asn Pro Leu Val Arg Gln Leu Gln Glu Lys Leu Pro Gly Gln Ser His
           355                 360                 365
   ttc agg tct gtg ctg aca gat gac tgc atg cgc gtc aag ggc agc gac      1152
   Phe Arg Ser Val Leu Thr Asp Asp Cys Met Arg Val Lys Gly Ser Asp
       370                 375                 380
   ggc tcc atc tgg gcg ctg ggc gac gcc gcc acc att gac cag ccc aag      1200
   Gly Ser Ile Trp Ala Leu Gly Asp Ala Ala Thr Ile Asp Gln Pro Lys
   385                 390                 395                 400
   gcg ctg gac tat gcc gag cag ctg ttt gag cag gcc gac acc aac cgc      1248
   Ala Leu Asp Tyr Ala Glu Gln Leu Phe Glu Gln Ala Asp Thr Asn Arg
                   405                 410                 415
   gat ggc cgc cta agc ttg gag gag ctg cgg gtg ctg ctc aac acc gca      1296
   Asp Gly Arg Leu Ser Leu Glu Glu Leu Arg Val Leu Leu Asn Thr Ala
               420                 425                 430
   tcc aag gag ttc agc cac ctg gag gag cac gcg cgc ttc ctg gac agc      1344
   Ser Lys Glu Phe Ser His Leu Glu Glu His Ala Arg Phe Leu Asp Ser
           435                 440                 445
   caa acc ggc gtc aag cgc ttc ggc ggc ctg gtc gcc aag tcg ctc agt      1392
   Gln Thr Gly Val Lys Arg Phe Gly Gly Leu Val Ala Lys Ser Leu Ser
       450                 455                 460
   ccg gcg gat gcg gcg gcg gcg gcg gcc agc aac agc agc cag ccc ttt      1440
   Pro Ala Asp Ala Ala Ala Ala Ala Ala Ser Asn Ser Ser Gln Pro Phe
   465                 470                 475                 480
   gcg gtg ctg ctg gac ggc aat acg gag atc tcc aag gag cag ttc aag      1488
   Ala Val Leu Leu Asp Gly Asn Thr Glu Ile Ser Lys Glu Gln Phe Lys
                   485                 490                 495
   gac att ctt ggc aag gtg gac aag ggc ctt cgc gcg ctg cct gcc acg      1536
   Asp Ile Leu Gly Lys Val Asp Lys Gly Leu Arg Ala Leu Pro Ala Thr
               500                 505                 510
   gcg cag gtg gcc aac cag cag ggc aag tac ctg gcg gcg gtg ttt gcg      1584
   Ala Gln Val Ala Asn Gln Gln Gly Lys Tyr Leu Ala Ala Val Phe Ala
           515                 520                 525
   ggc aac cgc gtc acg ggg gcc ccg gag ctg gac gca gcg ctg gcg gac      1632
   Gly Asn Arg Val Thr Gly Ala Pro Glu Leu Asp Ala Ala Leu Ala Asp
       530                 535                 540
   aag atc aag ccc ttc agg tac ttc cac aag ggc tcg gcc gcc tac gtg      1680
   Lys Ile Lys Pro Phe Arg Tyr Phe His Lys Gly Ser Ala Ala Tyr Val
   545                 550                 555                 560
   ggc agc gac aag gcc gtg ttc gac ctg ccc aag ttc ggg ccg ctc acg      1728
   Gly Ser Asp Lys Ala Val Phe Asp Leu Pro Lys Phe Gly Pro Leu Thr
                   565                 570                 575
   ggc acg ggc gcg ggc ttt gtg tgg aag agc tac gag acg atg tcg cag      1776
   Gly Thr Gly Ala Gly Phe Val Trp Lys Ser Tyr Glu Thr Met Ser Gln
               580                 585                 590
   ttc tcc ttc cgc aac cag tgt ctg gtc gcc gcc gac tgg ctg cgc acc      1824
   Phe Ser Phe Arg Asn Gln Cys Leu Val Ala Ala Asp Trp Leu Arg Thr
           595                 600                 605
   aag atc ttt ggc cgc gac atc tcg cga gtg tga                          1857
   Lys Ile Phe Gly Arg Asp Ile Ser Arg Val
       610                 615
<210> 4
   <211> 618
   <212> PRT
   <213> Chlamydomonas reinhardtii
<400> 4
   Met Asn Met Leu Leu Gln Gln Gln Lys Leu Ala Ala Gly Cys Lys Gln
   1               5                   10                  15
   Arg Ser Val Ala Gln Pro Ser Arg Gly Cys Val Ala Ala His Thr Gly
               20                  25                  30
   Leu Arg Ser Gly Arg Val Ala Ser Arg Gln Arg Ser Val Thr Thr Ala
           35                  40                  45
   Val Met Thr Pro Pro Ala Lys Ser Glu Ser Ser Ser Pro Val Tyr Thr
       50                  55                  60
   Thr Met Ser Leu Asp Gly Gln Asn Leu Lys Thr Ala Lys Pro Arg Leu
   65                  70                  75                  80
   Val Val Leu Gly Ser Gly Trp Gly Ala Met Ser Phe Leu Lys Ala Leu
                   85                  90                  95
   Pro Thr Ser Ile Ser Ser Thr Tyr Glu Leu Ile Val Val Ser Pro Arg
               100                 105                 110
   Asn Tyr Phe Leu Tyr Thr Pro Leu Leu Pro Ala Val Ala Thr Gly Thr
           115                 120                 125
   Met Glu Glu Arg Ser Ile Val Glu Pro Val Arg Asn Phe Ile Val Gly
       130                 135                 140
   Lys Gly Glu Phe Tyr Glu Ala Leu Cys Lys Asp Ile Asp Pro Val Ala
   145                 150                 155                 160
   Lys Glu Leu Val Cys Cys Phe Pro Glu Asp Ala Gly Leu Asp Ser Ala
                   165                 170                 175
   Cys Phe Lys Met Ser Tyr Asp Val Leu Val Met Ala Val Gly Ser Val
               180                 185                 190
   Asn Asn Thr Phe Gly Ile Lys Gly Val Asp Gln Tyr Cys Phe Tyr Phe
           195                 200                 205
   Lys Ser Ile Glu Asp Ala Asn Arg Leu Arg Ser Arg Val Ser Glu Cys
       210                 215                 220
   Phe Glu Arg Ala Ala Leu Pro Ala Thr Pro Glu Glu Glu Arg Lys Lys
   225                 230                 235                 240
   Leu Leu Thr Phe Val Val Val Gly Gly Gly Pro Thr Gly Val Glu Val
                   245                 250                 255
   Ala Ala Glu Leu Tyr Asp Met Ile Glu Glu Asp Leu Ser Lys Leu Tyr
                   260                 265                 270
   Pro Asn Leu Val Lys Asp Val Ser Ile Gln Val Val Glu Leu Met Asp
               275                 280                 285
   His Val Leu Ser Thr Tyr Asp Arg Ala Ile Ser Leu Tyr Thr Ala Glu
       290                 295                 300
   Gln Phe Lys Arg Ala Gly Ile Lys Leu Val Leu Asn Ser Arg Val Ala
   305                 310                 315                 320
   Ser Val Glu Asp Gly Val Val Arg Val Val Asn Lys Ala Asn Glu Ser
                   325                 330                 335
   Val Asp Ile Lys Phe Gly Ala Cys Val Trp Ala Thr Gly Ile Ala Met
               340                 345                 350
   Asn Pro Leu Val Arg Gln Leu Gln Glu Lys Leu Pro Gly Gln Ser His
           355                 360                 365
   Phe Arg Ser Val Leu Thr Asp Asp Cys Met Arg Val Lys Gly Ser Asp
       370                 375                 380
   Gly Ser Ile Trp Ala Leu Gly Asp Ala Ala Thr Ile Asp Gln Pro Lys
   385                 390                 395                 400
   Ala Leu Asp Tyr Ala Glu Gln Leu Phe Glu Gln Ala Asp Thr Asn Arg
                   405                 410                 415
   Asp Gly Arg Leu Ser Leu Glu Glu Leu Arg Val Leu Leu Asn Thr Ala
               420                 425                 430
   Ser Lys Glu Phe Ser His Leu Glu Glu His Ala Arg Phe Leu Asp Ser
           435                 440                 445
   Gln Thr Gly Val Lys Arg Phe Gly Gly Leu Val Ala Lys Ser Leu Ser
       450                 455                 460
   Pro Ala Asp Ala Ala Ala Ala Ala Ala Ser Asn Ser Ser Gln Pro Phe
   465                 470                 475                 480
   Ala Val Leu Leu Asp Gly Asn Thr Glu Ile Ser Lys Glu Gln Phe Lys
                   485                 490                 495
   Asp Ile Leu Gly Lys Val Asp Lys Gly Leu Arg Ala Leu Pro Ala Thr
               500                 505                 510
   Ala Gln Val Ala Asn Gln Gln Gly Lys Tyr Leu Ala Ala Val Phe Ala
           515                 520                 525
   Gly Asn Arg Val Thr Gly Ala Pro Glu Leu Asp Ala Ala Leu Ala Asp
       530                 535                 540
   Lys Ile Lys Pro Phe Arg Tyr Phe His Lys Gly Ser Ala Ala Tyr Val
   545                 550                 555                 560
   Gly Ser Asp Lys Ala Val Phe Asp Leu Pro Lys Phe Gly Pro Leu Thr
                   565                 570                 575
   Gly Thr Gly Ala Gly Phe Val Trp Lys Ser Tyr Glu Thr Met Ser Gln
               580                 585                 590
   Phe Ser Phe Arg Asn Gln Cys Leu Val Ala Ala Asp Trp Leu Arg Thr
           595                 600                 605
   Lys Ile Phe Gly Arg Asp Ile Ser Arg Val
       610                 615
<210> 5
   <211> 27
   <212> DNA
   <213> Artificial
<220>
   <223> N2Ag.mfe.F
<400> 5
   cgccaattga tgcaagaaca tcatgtt 27
<210> 6
   <211> 48
   <212> DNA
   <213> Artificial
<220>
   <223> N2Ag.6His.PstR
<400> 6
   aaaactgcag tcaatgatga tgatgatgat gggcctcgtc cttcagcg 48
<210> 7
   <211> 18
   <212> DNA
   <213> Artificial
<220>
   <223> PSD80.F
<400> 7
   gagctgttga caattaat 18
<210> 8
   <211> 18
   <212> DNA
   <213> Artificial
<220>
   <223> PSD80.R
<400> 8
   aggacgggtc acacgcgc 18
<210> 9
   <211> 17
   <212> DNA
   <213> Artificial
<220>
   <223> N2Ag.307.F
<400> 9
   tggccaccgg cgcgcgt 17
<210> 10
   <211> 18
   <212> DNA
   <213> Artificial
<220>
   <223> N2Ag.650.f
<400> 10
   tgcgaaggaa gcgcttga 18
<210> 11
   <211> 17
   <212> DNA
   <213> Artificial
<220>
   <223> N2Ag.901.R
<400> 11
   ttcctgattg accgcgg 17
<210> 12
   <211> 27
   <212> DNA
   <213> Artificial
<220>
   <223> ndhAgTu.F
<400> 12
   tcccccggga tgcaagaaca tcatgtt 27
<210> 13
   <211> 27
   <212> DNA
   <213> Artificial
<220>
   <223> ndhAgTu.R
<400> 13
   ccgcaattgt caggcctcgt ccttcag 27
<210> 14
   <211> 24
   <212> DNA
   <213> Artificial
<220>
   <223> N2Ag.NcoI
<400> 14
   atggaagaac atcatgttgt cgtc 24
<210> 15
   <211> 30
   <212> DNA
   <213> Artificial
<220>
   <223> N2Ag.XbaI
<400> 15
   ccgtctagat caggcctcgt ccttcagcgt 30
<210> 16
   <211> 21
   <212> DNA
   <213> Artificial
<220>
   <223> N2Ag.E203Q.R
<400> 16
   agggccggcc tgcacaagca a 21
<210> 17
   <211> 21
   <212> DNA
   <213> Artificial
<220>
   <223> N2Ag.E203Q.F
<400> 17
   ttgcttgtgc aggccggccc t 21
<210> 18
   <211> 24
   <212> DNA
   <213> Artificial
<220>
   <223> N2Cr2.F
<400> 18
   atgcatagcc ttgatggcca aaac 24
<210> 19
   <211> 21
   <212> DNA
   <213> Artificial
<220>
   <223> N2Cr2.R
<400> 19
   tcacactcgc gagatgtcgc g 21
<210> 20
   <211> 50
   <212> DNA
   <213> Artificial
<220>
   <223> F-EcoRI
<400> 20
   cggaattcat gcatcatcat catcatcatc atagccttga tggccaaaac 50
<210> 21
   <211> 30
   <212> DNA
   <213> Artificial
<220>
   <223> R-SmaI
<400> 21
   tcccccgggt cacactcgcg agatgtcgcg 30
<210> 22
   <211> 30
   <212> DNA
   <213> Artificial
<220>
   <223> N2Ag.XhoI
<400> 22
   cggctcgaga tggccgccgt cattgccaag 30
<210> 23
   <211> 48
   <212> DNA
   <213> Artificial
<220>
   <223> TP.ndh.R
<400> 23
   gacgacaaca tgatgttctt gcatctggtt ggcctgagcc ggggcagc 48
<210> 24
   <211> 48
   <212> DNA
   <213> Artificial
<220>
   <223> TP.ndh.F
<400> 24
   gctgccccgg ctcaggccaa ccagatgcaa gaacatcatg ttgtcgtc 48
<210> 25
   <211> 48
   <212> DNA
   <213> Artificial
<220>
   <223> rbcS2.3'.R
<400> 25
   gctcagatca acgagcgcct ccattcaggc ctcgtccttc agcgtctc 48
<210> 26
   <211> 48
   <212> DNA
   <213> Artificial
<220>
   <223> rbcS2.3'.F
<400> 26
   gagacgctga aggacgaggc ctgaatggag gcgctcgttg atctgagc 48
<210> 27
   <211> 27
   <212> DNA
   <213> Artificial
<220>
   <223> N2Ag.KpnI
<400> 27
   cggggtaccc tgcaaatgct gtctcca 27